# EUROPEAN PATENT APPLICATION

(11) **EP 4 567 819 A1**
(43) Date of publication of application: **11.06.2025**
(21) Application number: 24218226.9
(22) Date of filing: 08.12.2024
(51) Int. Cl.: G16C 60/00

(54) **APPARATUS AND COMPUTER-IMPLEMENTED METHOD FOR PRODUCING A MOLECULARLY IMPRINTED POLYMER NANOPARTICLE BASED ON A MACHINE LEARNING ALGORITHM**

(30) Priority: 08.12.2023 LU 505728
(71) Applicant: Christian-Albrechts-Universität zu Kiel, 24118 Kiel (DE)
(72) Inventor: Altintas, Zeynep, 24143 Kiel (DE); Moldovean-Cioroianu, Nastasia Sanda, 24143 Kiel (DE)
(74) Representative: Stüven, Ralf

(57) **Abstract**

A computer-implemented method for producing a multifunctional molecularly imprinted polymer nanoparticle recipes based on a machine learning algorithm, wherein the molecularly imprinted polymer nanoparticle comprises a template molecule, one or more (up to 3) functional monomers and cross-linker, wherein the computer-implemented method comprises: importing a programming library; receiving an input dataset; pre-processing an input dataset, wherein the defined dataset comprises data of functional monomers, template molecules and cross-linkers; including data on the amino acid types within the template sequences; classifying functional monomers and cross-linker based on the pre-defined dataset and selection criteria; predicting functional monomers, cross-linker, and their ratios for new template sequences; outputting data of the followings: best/optimal functional monomers, cross-linker and their ratios on a display means, for a given template molecule.

## Description

### FIELD

The present invention is in the realm of bio-mimetic materials and therapeutic strategies. In particular, the present invention relates to an apparatus and a computer-implemented method for producing a molecularly imprinted polymer nanoparticle based on a machine learning algorithm.

### BACKGROUND

The term "molecularly imprinted polymer nanoparticle" (MIP-NP) relates to a nanoparticle comprising at least one molecularly imprinted polymer (MIP), preferably a plurality of molecularly imprinted polymers (MIPs) (see, for example, Fresco-Cala B, Batista AD, Cárdenas S. Molecularly Imprinted Polymer Micro- and Nano-Particles. A review. Molecules. 2020 Oct 15;25(20):4740. doi: 10.3390/molecules25204740).

Molecularly imprinted polymers (MIPs) are prepared by synthesizing a three-dimensional polymer matrix around a template molecule. After the template molecule is removed, a MIP is obtained having cavities that are complementary to parts of the target molecule, thus forming selective recognition sites for the target molecule. The MIP obtained is able to specifically bind the template molecule non-covalently. The template molecule may be, for example, a biomolecule like a protein, a fragment of a protein, a nucleic acid, e.g., a messenger RNA molecule, or a fragment of a nucleic acid, or an enzyme, or a virus capsid. A fragment of a biomolecule preferably comprises a characteristic structure of the template molecule, for example, in case of e.g. a protein, a helix structure, a loop structure, a beta-sheet structure or any other structure comprising a stretch of amino acids forming a recognizable structure. In case of nucleic acids as a template, a fragment may preferably comprise a nucleotide sequence forming a loop structure, a stem structure or helix structure.

MIP-NP can be used for many applications including biosensing, diagnosis, and drug delivery platforms, for example, in the cystic fibrosis treatment. The term "cystic fibrosis" (CF, ICD-10 code E84, 2024 edition), also called "Mucoviscidosis", refers to a genetic disorder inherited in an autosomal recessive manner. In cystic fibrosis, exocrine (secretory) glands produce abnormally thick mucus. It is caused by mutations in both copies of the gene for the cystic fibrosis transmembrane conductance regulator (CFTR) protein. Cystic fibrosis can, for example, be caused by a frameshift mutation resulting in the introduction of a premature stop codon (see, e.g., lannuzzi MC, Stern RC, Collins FS, Hon CT, Hidaka N, Strong T, Becker L, Drumm ML, White MB, Gerrard B, Dean, M., 1991, Two frameshift mutations in the cystic fibrosis gene, Am J Hum Genet. 48(2):227-31, PMID: 1990834; PMCID: PMC1683026).

The present invention aims to provide an apparatus and a method for manufacturing multifunctional MIP-NP with potential applications that go beyond CF. This multifunctionality arises (upon design) from the potential MIP-NPs' capabilities to: (i) target specific structures (proteins, enzymes, viruses) from complex media, (ii) deliver the payload (e.g. drug and/or gene/mRNA), and (iii) treat diseases - that were initially considered in MIP-NPs' design processes - by releasing the payload to targeted cells/affected regions.

The present invention not only aligns with the industry's ongoing shift towards highly specific and customizable biomimetic polymers, but also offers new opportunities for research, development, and manufacturing of advanced biomimetic, smart materials. As a result, the present invention offers to enhance patient outcomes and open new avenues for commercial success in healthcare and biotechnology industries.

Conventional methods struggle with the complexity of target molecule structures and the vast range of functional monomer combinations, often leading to sub-optimal MIP-NP performance and inefficient design processes. The present invention mitigates these limitations.

Conventional MIP-NP design methods demand significant time and resources, whereas the ML algorithm of the present invention revolutionizes the process by delivering precise MIP-NP formulations for specific template molecules in seconds, drastically improving research efficiency and accuracy.

### SUMMARY OF THE INVENTION

In a first aspect, the present invention provides a computer-implemented method for producing a multifunctional molecularly imprinted polymer nanoparticle (MIP-NP) based on a machine learning algorithm, wherein the molecularly imprinted polymer nanoparticle comprises a template molecule, a functional monomer and a cross-linker.

The computer-implemented method comprises: receiving an input dataset; importing a programming library; processing a pre-defined dataset, wherein the pre-defined dataset comprises data of functional monomers, template molecules and cross-linkers; finding a functional monomer, a template molecule and a cross-linker, wherein the functional monomer, the template molecule and the cross-linker have a maximum number of H-bonds (hydrogen bonds) and a minimum value of total interaction energy based on the input dataset; and outputting data of at least one of the followings: the functional monomer, the template molecule and the cross-linker on a display means.

It can be understood that, the sequence of precedence of the step of receiving an input dataset and the step of importing a programming library can be interchanged.

In a preferred embodiment of the present invention, the computer-implemented method comprises: importing a programming library; receiving an input dataset; processing a pre-defined dataset, wherein the pre-defined dataset comprises data of functional monomers, template molecules and cross-linkers; finding a functional monomer, and a cross-linker, wherein the functional monomer, and the cross-linker are predicted on new, unseen template molecule based on a pre-defined selection criteria; and outputting data of at least one of the followings: the functional monomer, the template molecule, the cross-linker, and a ratio of the functional monomer and the cross-linker specific for the template molecule, on a display means.

It can be understood that, the input dataset is to be selected based on an intended use of the MIP-NP, and in this case the MIP-NP developed via the computer-implemented method are suited for biosensing, diagnosis, and targeted payload delivery.

It can also be understood that, the three components for the MIP-NP design: functional monomer, template molecule and cross-linker are interdependent. When the computer-implemented method identifies a suitable functional monomer for the template, e.g. an amino acid sequence, based on the input dataset, for example the training dataset, it concurrently identifies the crosslinking agent and preferably also the best suitable template-monomer-crosslinker ratios, for developing/designing the most suitable MIP-NP recipes based on the provided template (e.g. amino acid sequence) by the users. In a preferred embodiment, the criterion of the most suitable MIP-NP recipe comprises of increased gyration values, proper minimum distances between MIP components, a maximum number of H-bonds and a minimum value of total interaction energy. Preferably, the minimum value of total interaction energy refers to a most negative value of total interaction energy.

In an embodiment of the present invention, each input dataset and optionally any derived data calculated by the machine learning algorithm based on each input dataset can be stored into the pre-defined dataset.

In an embodiment of the present invention, before performing prediction tasks, the algorithm classifies the MIP components based on their performance, which is strongly correlated with feature correlations and selection criteria. This performance classification is then stored in a new column labeled 'Performance' within the CSV dataset. Finally, the ML model uses this 'Performance' column to predict outcomes for new MIP formulations on previously unseen template sequences.

In an embodiment of the present invention, selection criteria used for classification tasks consists of (i) physico-chemical properties of the input template sequences by user, (ii) gyration values of MIP complexes, (iii) minimum distances between MIP components (template and functional monomers), (iv) H-bonds between template and functional monomers, (v) total interaction energies between template-monomers-crosslinkers. Based on this selection criteria, the classification of functional monomers and crosslinkers are being performed. Afterwards, this classification task is directly used in the subsequent prediction task, where the best performing MIP-NP components (functional monomers, cross-linkers, and their ratios) are predicted for new, unseen template sequences prompted by the user.

It can be understood that the selection criteria for MIP-NP classification incorporate physico-chemical properties, interaction metrics, and weighted relevance of interdependent features, ensuring context-sensitive and precise predictions.

In an embodiment of the present invention, in the step of outputting data of at least one of the followings: the functional monomer, the template molecule, the cross-linker, and a ratio of the functional monomer and the cross-linker on a display means, multiple functional monomers and their ratios with the cross-linker and the cross-linker are outputted on the display means.

In an embodiment of the present invention, wherein after the step of processing a pre-defined dataset, the computer-implemented method further comprises: prompting a user on the display means to add data of a new functional monomer into the pre-defined dataset via an input means.

In the above embodiment, preferably the data of the new functional monomer comprises at least one of the followings: an index number, a name, a type, a docking value, an epitope sequence, a contact number, a solvent-accessible surface area, a H-bonds number, and a total interaction energy of the new functional monomer.

The template molecules are epitopes in this embodiment. The term "epitope sequence" relates to a specific amino acid sequence extracted from whole target molecules (e.g., proteins, enzymes, virus coats).

The term "docking value" relates to a numerical score or energy value assigned upon molecular docking simulations, reflecting on the binding affinity between the monomers and template molecules (the more negative score, the higher the affinity).

The term "contact number" relates to a number of interactions between functional monomers and template molecules. MIP components may, for example, be considered in contact if the respective distances between alpha carbon atoms are below the cutoff distance of 0.6 nm.

The term "solvent accessible surface area" relates to the surface area of the template molecule that is highly exposed/accessible to solvent.

The term "hydrogen bonds" relates to attractive interactions between hydrogen atoms bonded to strongly electronegative atoms that exist in the vicinity of other electronegative atoms with a lone pair of electrons (the distance between donor and acceptor atoms is usually 0.27-0.35 nm).

The term "total interaction energy" relates to an overall energy associated with the interactions between monomers, templates, and cross-linkers. It includes contributions from van der Waals forces (Lennard-Jones potentials) and electrostatic contributions (Coulomb potentials). Namely, it quantifies the net effect of all forces influencing the interactions between molecules within a system.

In an embodiment of the present invention, after the step of finding a functional monomer, a template molecule and a cross-linker, the computer-implemented method further comprises: storing the data of the at least one of the functional monomer, the template molecule and the cross-linker to a new column of the pre-defined dataset.

In an embodiment of the present invention, wherein the data of the at least one of the template molecule, the functional monomer and the cross-linker comprises at least one of the followings: an index number, a name, a type, a docking value, an epitope sequence, a contact number, a solvent-accessible surface area, a H-bonds number, and a total interaction energy of the at least one of the template molecule, the functional monomer and the cross-linker.

In a preferred embodiment of the present invention, wherein the computational data/datapoints of the at least one of the template molecule, the functional monomer and the cross-linker comprises at least one of the followings: an index number for each component and complex, an abbreviated name, a type (acidic, basic, neutral), a docking value, an epitope sequence, a solvent-accessible surface area, a H-bonds number, a gyration value, a minimum distance value and a total interaction energy representative for the interactions between the tested template molecules, the functional monomers and the cross-linkers.

In an embodiment of the present invention, wherein the experimental datapoints of the at least one of the template molecule, the functional monomer and the cross-linker comprises at least one of the followings: Fourier transform infrared spectroscopy (FTIR) fingerprints and wavenumbers, hydrodynamic particle sizes at both 25°C and 38°C, and the limit of detection values extracted from sensor assays.

In an embodiment of the present invention, both computational and experimental datapoints are correlated and integrated in the same input dataset.

In an embodiment of the present invention, wherein in the step of finding a functional monomer and a cross-linker, a machine learning classifier module is used. In the above embodiment, preferably, Random Forest classifier is used.

The present invention can most suitably be considered as a classification task. Random Forest classifier belongs to the ensemble learning sub-category, and it combines the predictions of multiple decision trees (significantly reducing the over-fitting) to make accurate predictions. Random Forest classifier is thus suitable for the present invention and has shown good performance. In an embodiment, the Random Forest classifier to be used consists of 400 decision tress, with a maximum depth per tree of 10, random state parameter of 135, and a test size of 0.3 representing that 30% of the input dataset is used for testing.

It can be understood that, subsequent to the classification of MIP components tested and integrated into the dataset, the present invention also aims to predict new MIP formulations for unseen template/target sequences.

In an embodiment of the present invention, wherein all the steps of the computer-implemented method are repeated for all data in a training dataset and a testing dataset for training. It can be understood that, the training dataset and the testing dataset are received as the input dataset by the computer-implemented method of the present invention; and/or the training dataset and the testing dataset belong to the pre-defined dataset.

It can be understood that, in the above embodiment, dataset is split into the training dataset and the testing dataset for training purpose. For example, the computer-implemented method is in the form of a machine learning program. To train and assess the performance of the machine learning program, and make sure that it can perform well to novel and unseen data (such as new template molecules), it is fundamental to train and test the machine learning program based on training dataset and testing dataset.

Preferably, the dataset is split into training and testing sets, with 70% of the data allocating to training and 30% of the data allocated to testing, thereby ensuring a robust evaluation of the model's performance.

In an embodiment of the present invention, a confusion matrix is calculated to assess a prediction accuracy of the computer-implemented method.

It can be understood that, in order to assess the prediction accuracy of the computer-implemented method, marks can be calculated for grading the prediction performance. According to the above embodiment, confusion matrix is calculated for this purpose based on the number of "true positive", "true negative", "false positive" and "false negative" of the prediction results.

In an embodiment of the present invention, wherein the input dataset comprises data relating to at least two of the followings: a template molecule, a functional monomer and a cross-linker; preferably, input dataset comprises a binding affinity.

It can be understood that, the input dataset is selected based on an intended use of the MIP-NP. According to the above embodiment, the input dataset comprises data relating to at least two of the components of the MIP-NP. For example, binding affinity reflects not only the functional monomer but also the template molecule, since template molecule and function monomer interact with each other. Based on the input dataset, i.e., a condition that reflects at least two of the components, the present invention conducts the task of finding the optimal recipe of MIP-NP.

In an embodiment of the present invention, the data of the input dataset and/or the pre-defined dataset comprise computation data collected from a computational study and/or experimental data collected from an experimental study; and/or the data of the input dataset and/or pre-defined dataset comprise numerical and categorical variables, wherein numerical variables represent continuous or discrete numerical data, and categorical variables represent qualitative data, such as labels or categories. Therefore, the data in the input dataset and/or pre-defined dataset altogether integrate a diverse range of information types, a more accurate "simulation" of the real-world conditions can be achieved, and the inclusion of different types of data points allows for flexible and multidimensional data analysis.

In an embodiment of the present invention, the input dataset comprises an amino acid sequence. For example, the computer-implemented method received an amino acid sequence input by the user. Preferably, the amino acid sequence input by the user has a maximum of 20 amino acid residues, wherein each amino acid residue is represented using the standardized one-letter code system for amino acids.

In the above embodiment, preferably, categorical variables are converted into a numerical format, for example by LabelEncoder tool from the sklearn.preprocessing library.

In an embodiment of the present invention, the functional monomer is temperature-responsive or pH-responsive or chemically-responsive for the molecularly imprinted polymer nanoparticle to release an active ingredient to a target. These features unlock new horizons for crafting MIP-NP carriers with the ability to deliver an active ingredient to their target.

In an embodiment of the present invention, the template molecule is DDGCEPCGNDT peptide, extracted from the DNase-I structure.

In an embodiment of the present invention, an active ingredient to be delivered to a target by the molecularly imprinted polymer nanoparticle is a drug or a mRNA. The tailor-made MIP-NPs enhance drug delivery systems, ensuring that medications or mRNAs reach their intended targets with greater efficiency, while minimizing side effects and optimizing the treatment outcomes.

In an embodiment of the present invention, the target is a cell. Preferably a lung cell. In this embodiment, the tailor-made MIP-NP carriers can be effectively used for CF treatment, providing advanced strategies, aiding in the removal of excess mucus from the CF airways.

In an embodiment of the present invention, the computer-implemented method is based on supervised learning.

In an embodiment of the present invention, all features in the dataset are (initially) scaled for standardization purposes to ensure that all variables/datapoints contribute equally to the model, preventing those with larger numerical ranges from dominating the final predictions.

The present invention according to the first aspect allows a design of MIP-NPs using advanced computational methods, bringing a substantial enhancement in the precision of MIP-NPs' design. It has enabled the selection of the optimal MIP-NP recipe comprising functional monomer, template molecule and cross-linker. A rapid and robust method for highly specific and customizable detection and potential therapeutics is thereby achieved and new opportunities for research, development, and manufacturing of advanced biomimetic, smart materials are also provided.

In a second aspect, the present invention provides a method for producing a multifunctional molecularly imprinted polymer nanoparticle, wherein the molecularly imprinted polymer nanoparticle comprises a template molecule, a functional monomer and a cross-linker.

The method comprises: selecting an input dataset based on an intended use; inputting the input dataset via an input means; receiving a data of at least one of the followings: a template molecule, a functional monomer, a cross-linker and a template-monomer-crosslinker ratio on a display means, wherein the data is obtained according to any one of embodiments mentioned above; producing the molecularly imprinted polymer nanoparticle according to the data of at least one of the followings: the template molecule, the functional monomer and the cross-linker.

It can be understood that, the second aspect provides a different use of the same invention as described in the first aspect.

In an embodiment of the present invention, the method for producing a multifunctional molecularly imprinted polymer nanoparticle based on a machine learning algorithm comprises: selecting an input dataset based on an intended use; inputting the input dataset via an input means; receiving at least one of a template molecule, a functional monomer, a cross-linker and a template-monomer-crosslinker ratio on a display means, wherein the data are obtained according to a computer-implemented method for producing a multifunctional molecularly imprinted polymer nanoparticle based on a machine learning algorithm, and producing the molecularly imprinted polymer nanoparticle according to the data of at least one of the followings: the template molecule, the functional monomer, the cross-linker and the template-monomer-crosslinker ratio; wherein the computer-implemented method comprises the following: importing a programming library; receiving an input dataset; processing a pre-defined dataset, wherein the pre-defined dataset comprises data of functional monomers, template molecules and cross-linkers; finding a functional monomer, and a cross-linker, wherein the functional monomer, the template molecule and the cross-linker have a maximum number of H-bonds and a minimum value of total interaction energy based on the input dataset; and outputting at least one of the followings data: the functional monomer, the cross-linker and a ratio of the functional monomer and the cross-linker specific for the template molecule on the display means.

It can be understood that, the sequence of precedence of the step of receiving an input dataset and the step of importing a programming library can be interchanged.

In an embodiment of the present invention, wherein after the step of processing an input dataset comprising both computational and experimental data, the method further comprises converting a pre-defined dataset into a machine-readable format. Preferably the machine-readable format is CSV format.

In a third aspect, the present invention provides an apparatus for producing a multifunctional molecularly imprinted polymer nanoparticle based on a machine learning algorithm, wherein the molecularly imprinted polymer nanoparticle comprises a template molecule, a functional monomer and a cross-linker.

The apparatus comprises: a receiving means configured to receive an input dataset, an input means configured for a user to input data of a new functional monomer and/or the input dataset, a processing means configured to import a programming library, and/or process a pre-defined dataset, and/or find a functional monomer, a template molecule and a cross-linker, wherein the functional monomer, the template molecule and the cross-linker have a maximum number of H-bonds and a most negative value of total interaction energy based on the input dataset, and a display means configured to screen a pre-defined dataset and/or output data of at least one of the followings: the template molecule, the functional monomer and the cross-linker.

In a further aspect, the present invention provides an electronic device, wherein the electronic device comprises a processor and a memory, the memory stores a computer-executable instruction executable by the processor, the processor is configured to execute the computer-executable instruction so as to implement a computer-implemented method for producing a multifunctional molecularly imprinted polymer nanoparticle based on a machine learning algorithm, wherein the molecularly imprinted polymer nanoparticle comprises a template molecule, a functional monomer and a cross-linker, according to any one of embodiments mentioned above.

In a further aspect, the present invention provides a computer-readable storage medium, wherein the computer-readable storage medium stores a computer-executable instruction, that, when being executed by a processor, causes the processor to implement a computer-implemented method for producing a multifunctional molecularly imprinted polymer nanoparticle based on a machine learning algorithm, wherein the molecularly imprinted polymer nanoparticle comprises a template molecule, a functional monomer and a cross-linker, according to any one of embodiments mentioned above.

In a further aspect, the practical implication of the invention relates to the design of a molecularly imprinted polymer, MIP, or a molecularly imprinted polymer nanoparticle, MIP-NP, the MIP or MIP-NP that may carry an mRNA molecule. Preferably, the MIP or MIP-NP specifically binds the mRNA molecule. Further preferred, the mRNA is an mRNA coding for functional gene product of a gene of interest, or a fragment thereof. A fragment is preferably a functional fragment, i.e., a fragment having at least partly the function of the full functional gene product. Particularly preferred the MIP or MIP-NP specifically binds an mRNA coding for a functional gene product, e.g. a protein, the absence or malfunction of which causes a disease in a human. In a preferred embodiment of the invention the MIP or MIP-NP specifically binds an mRNA coding for a functional CFTR gene.

In a preferred embodiment of this aspect of the invention, the MIP or MIP-NP is designed to target specific cells, e.g. cells of a specific tissue or organ, in the human body, e.g. lung cells, that consist of the targeted template molecule, e.g., CFTR protein. In this embodiment, the MIP or MIP-NP of the invention is designed in such a manner that the functional mRNA gene is specifically, or at least predominantly, delivered via the MIP or MIP-NP acting as a carrier to a human tissue or organ of interest, e.g. to lung cells of a patient suffering from cystic fibrosis. The functional monomer in this regard can be temperature-responsive, so as to release the cargo molecule (functional mRNA gene) to the target.

In a preferred embodiment of the invention the MIP or MIP-NP may, for example, be designed by the computer-implemented method in such a manner, that its performance is stimuli-responsive, i.e., responsive to physical and/or chemical and/or biological environmental stimuli.

The above-described MIP or MIP-NP of the invention can advantageously be used as a medicament for the treatment of a disease, for example, a heritable disease, in which, for example, the lack or malfunction of a protein, is causing the disease. The MIP or MIP-NP of the invention can be used to deliver an active agent or medicament to a target site in the body, for example, human body, e.g. to target cells like lung cells. In a further aspect, the invention thus also relates to a molecularly imprinted polymer, MIP, or a molecularly imprinted polymer nanoparticle, MIP-NP, the MIP or MIP-NP carrying non-covalently bound thereto an mRNA molecule, for use in treating cystic fibrosis.

In another aspect, the invention relates to a molecularly imprinted polymer, MIP, or a molecularly imprinted polymer nanoparticle, MIP-NP, the MIP or MIP-NP carrying non-covalently bound thereto a mucolytic agent, for example, a mucolytic enzyme, e.g. Dornase alfa (rhDNase I), for use in treating cystic fibrosis (see, for example, Shak S, Capon DJ, Hellmiss R, Marsters SA, Baker CL. Recombinant human DNase I reduces the viscosity of cystic fibrosis sputum. Proc Natl Acad Sci USA. 1990 Dec;87(23):9188-92. doi: 10.1073/pnas.87.23.9188). In a particularly preferred embodiment of the invention, the MIP or MIP-NP carries non-covalently bound thereto a recombinant human DNase I (rhDNasel; amino acid sequence see SEQ ID NO: 1; PDB Entry 4AWN).

Routes of administration depend on the targeted organ, tissue or cell. Preferred routs of administration are, for example, oral, e.g. in the form of a powder, or pulmonary delivery, e.g. in the form of a liquid aerosol, liquid bolus or dry powder inhalation. Pulmonary delivery, e.g. via inhalation as a liquid aerosol, is particularly preferred in the case of cystic fibrosis.

In a still further aspect, the invention also relates to a pharmaceutical composition comprising an effective amount of a MIP or MIP-NP of the invention as described above, and, if necessary or advantageous, a pharmaceutically acceptable carrier. A "pharmaceutically acceptable carrier" is any of the standard pharmaceutical carriers, and includes pharmaceutically acceptable excipients, diluents, fillers, disintegrants, lubricants and other agents that can function as a carrier or excipients. The term "pharmaceutically acceptable carrier" means a carrier that is useful in preparing a pharmaceutical composition that is generally safe and non-toxic, and includes carriers that are acceptable for veterinary use as well as for human pharmaceutical use. Such carriers can be solid, liquid, semisolid, or, in the case of an aerosol composition, gaseous. Pharmaceutical compositions are prepared in accordance with acceptable pharmaceutical procedures, such as described in Remington's Pharmaceutical Sciences, 17th edition, ed. Alfonoso R. Gennaro, Mack Publishing Company, Easton, Pa. (1985). Pharmaceutically acceptable carriers are those that are compatible with the other ingredients in the formulation and biologically acceptable. The pharmaceutical composition may be constituted into any form suitable for the mode of administration selected. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

It is particularly preferred that all the MIP or MIP-NPs of the invention described above are obtained or obtainable by the method of the invention.

In the following, the invention will be described in further detail by way of example only with reference to the accompanying figures.

### BRIEF DESCRIPTION OF THE DRAWINGS

Figure 1. Atomic root-mean-square fluctuations (RMSF) of input DNase-I structure (1st set of MD productions).
Figure 2. Atomic RMSF of DNase-I structure (2nd set of MD productions).
Figure 3. Molecular docking parameters.
Figure 4. Docked, solvated, and energy minimized input systems for 150 ns MD productions at 300 K temperature and 1 bar pressure (2 NIPAM systems - left, 37 NIPAM systems - right, template - 1, functional monomers - 2, solvent - 3, ions - 4).
Figure 5. Structural (radius of gyration - left) and solvent-related behavior (solvent exposure area - right) of the selected template molecule (DDGCEPCGNDT peptide) in interaction with 2 NIPAM system (light grey plot) vs. 37 NIPAM system (dark grey) at 300 K temperature.
Figure 6. Distribution of minimum distances (below 0.2 nm) for template-monomer interactions over 150 ns MD productions at 300 K temperature. Systems with 2 NIPAM (top) and 37 NIPAM (bottom) molecules were compared. x-axis = Minimum Distance [nm], y-axis = Frequency.
Figure 7. Comparison between H-bonds number for systems with 37 NIPAM molecules (at 300 K), when including different concentrations of BIS cross-linker. The systems without BIS were also considered for comparison.
Figure 8. Minimum distance analysis for systems with different BIS concentrations.
Figure 9. Total interaction energies at different temperatures for systems with distinctive BIS concentrations.
Figure 10. Schematic representation of the experimental approach of an embodiment of the present invention for MIP-NP synthesis targeting DNAse-I.
Figure 11. Hydrodynamic size distribution (left) and zeta potential (right) of synthesized MIP-NPs targeting DNAse-I, using DLS at 25°C vs. 38°C according to an embodiment of the present invention.
Figure 12. FT-IR spectra of synthesized MIP-NPs targeting DNAse-I.
Figure 13. Flow diagram of a computer-implemented method for producing a multifunctional molecularly imprinted polymer nanoparticle based on a machine learning algorithm according to an embodiment of the present invention.
Figure 14. Flow diagram of a method for producing a multifunctional molecularly imprinted polymer nanoparticle according to an embodiment of the present invention.
Figure 15. Flow diagram showing a detailed workflow of an embodiment of the computer-implemented method for producing a multifunctional molecularly imprinted polymer nanoparticle based on a machine learning algorithm according to an embodiment of the present invention.
Figure 16(a) and 16(b). An example of categorical features and numerical features of an input dataset.
Figure 17. Computational workflow considered for T-FM complexes setup and simulations including both molecular docking and MD approaches. This workflow was used for generating all computational datapoints used in input dataset.
Figure 18. Input dataset features, alongside the required libraries, used for algorithm development. The 1-12 annotation in this figure refers to the earlier steps outlined in Figure 17.
Figure 19. Solid-phase synthesis method of the ML-based nanoMIP recipes (A-F), including the chemical structure of the monomer, crosslinker and dye monomer used in the synthesis process.

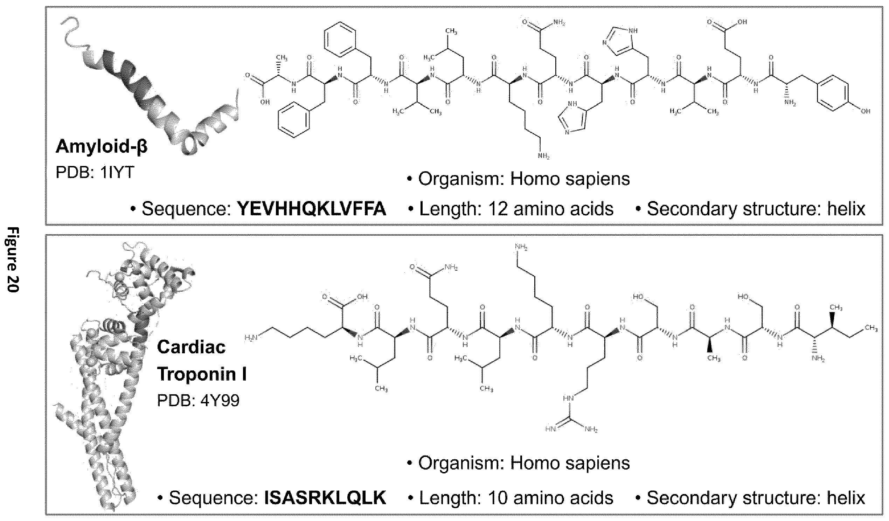

Figure 20. Characteristics of template molecules (amino acid sequences) used for the generation of new ML-based nanoMIP recipes. Template 1 (A) (PDB: 1IYT (Crescenzi, O., Tomaselli, S., Guerrini, R., Salvadori, S., D'Ursi, A.M., Temussi, P.A. and 1265 Picone, D. Solution structure of the Alzheimer amyloid β-peptide (1-42) in an apolar 1266 microenvironment. Eur J Biochem, (2002), 269: 5642-5648. DOI: 10.1046/j.1432¬1267 1033.2002.03271.x) was used as input sequence for designing nanoMIPs specific to amyloid-3, while template 2 (B) (PDB: 4Y99 (Takeda, S., Yamashita, A., Maeda, K. et al. Structure of the core domain of human 1262 cardiac troponin in the Ca2+-saturated form. Nature (2003), 424, 35-41. DOI: 1263 10.1038/nature01780) was used to design nanoMIPs targeting cardiac troponin-I epitope. Both epitopes of interest are highlighted in red.
Figure 21. Hydrodynamic sizes (A, D, G), stability in suspension (B, E, H), and fluorescence imaging of the gold screen-printed electrodes (GSPE) with immobilized nanoMIPs (C, F, I). NanoMIP1 corresponds to sub-figures A, B, C; nanoMIP2 to D, E, F and nanoMIP3 is illustrated in G, H, I.
Figure 22. Peptide rebinding studies (shown for concentration range between 100 nM - 2 µM) of all nanoMIP formulations (nanoMIP1: top, left; nanoMIP2: top right; and nanoMIP3: bottom). The plots show fittings against Langmuir, Freundlich, and Langmuir-Freundlich binding isotherms.

### DETAILED DESCRIPTION OF EXEMPLARY EMBODIMENTS

### Example 1

The following example describes the design of a molecularly imprinted polymer, MIP, for binding a recombinant human DNase I (rhDNase I) that can advantageously be used in the treatment of cystic fibrosis. This example, specifically for designing MIPs targeting DNAse-I, follows the step-wise workflow considered for all computational datapoints present in our input dataset used to train and test the ML algorithm.

### I. Computational section - MIPs design targeting DNAse-I

### 1. Template molecule selection

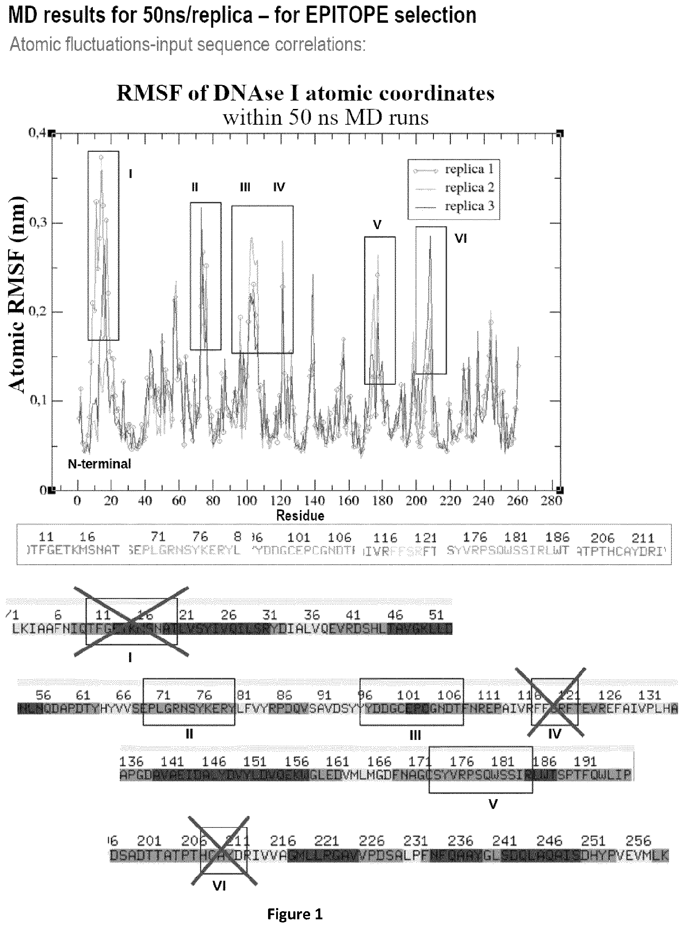

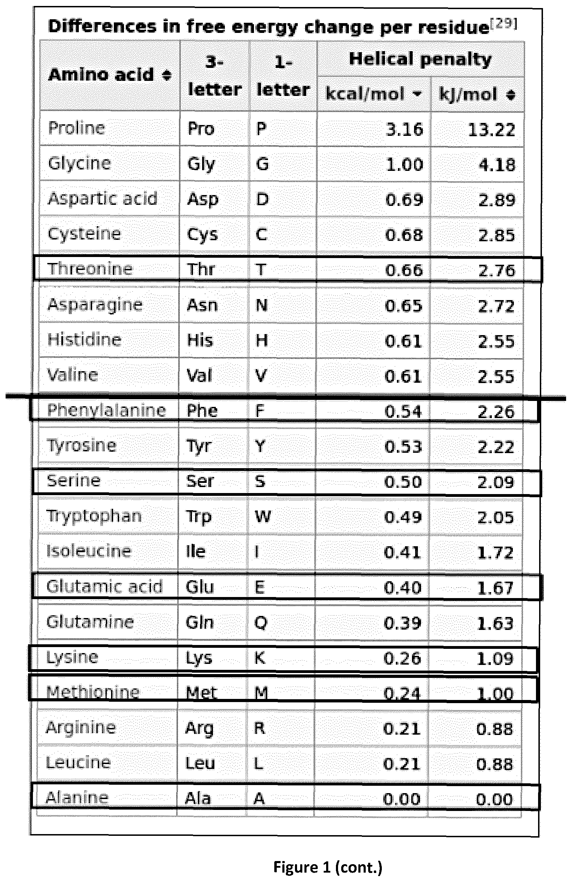

The PDB code (see Goetz Parsiegla, Christophe Noguere, Lydia Santell, Robert A. Lazarus, and Yves Bourne. The Structure of Human DNase I Bound to Magnesium and Phosphate Ions Points to a Catalytic Mechanism Common to Members of the DNase I-like Superfamily. Biochem. 2012, 51 (51), 10250-10258. DOI: 10.1021/bi300873f; PDB entry 4AWN ) of DNase-I was used for setting up the molecular dynamics (MD) system at 300K temperature and 1 bar pressure. The initial set of MD productions were carried out using Gromacs software package (Van Der Spoel D, Lindahl E, Hess B, Groenhof G, Mark mL, Berendsen HJ. GROMACS: fast, flexible, and free. J Comput Chem. 2005, 26(16):1701-18. DOI: 10.1002/jcc.20291) for 50 ns and in 3 replicas. Figure 1 shows the atomic root-mean-square fluctuations (RMSF) of input DNase-I structure upon MD productions.

Six potential epitopes, i.e., amino acid sequences, were identified as promising candidates for template molecule selection (see boxes I-VI in the upper right part of Figure 1, and the corresponding boxes I-VI for the RMSF data in the left part of Figure 1). Epitopes with high propensities for helical configurations, as well as those with short amino acid chains, were eliminated (boxes with crosses). Moreover, due to elevated structural and dynamical instability, potential for non-specific interactions and non-representative binding sites of the terminal ends (N-terminal domain of DNase-I), the focus was mainly oriented towards the residue ranges 60-120, and 180-210.

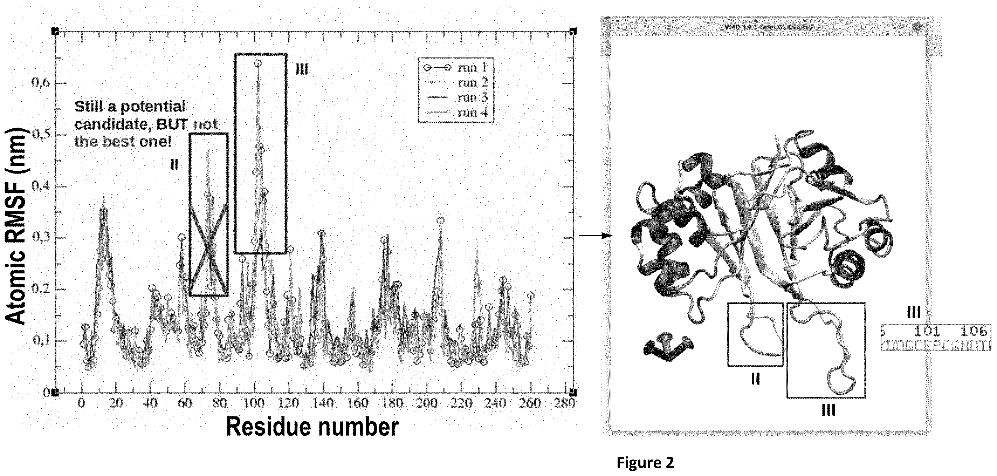

Epitopes II, III, and V (see boxes in Figure 1) were selected as the most suitable amino acid chains for significantly longer MD investigations of 350 ns, and in 4 replicas (see Figure 2 for the atomic RMSF of DNase-I structure upon the 2nd set of the MD productions).

Finally, based on the RMSF data obtained, epitope III was selected as a template molecule for MIP design, with the amino acid sequence: DDGCEPCGNDT (11 amino acid long; SEQ ID NO: 2).

### 2. Molecular docking between functional monomers and the selected template molecule

Subsequently, extensive molecular docking investigations were carried out with 4 potential functional monomers docked against the selected template molecule (DDGCEPCGNDT, SEQ ID NO: 2).

The following functional monomers of interest were:
˙N-Isopropylacrylamide (NIPAM)
˙N-(3-Aminopropyl)methacrylamide (APMA)
˙Acrylic acid (AcA)
˙N-phenylacrylamide (NPA)

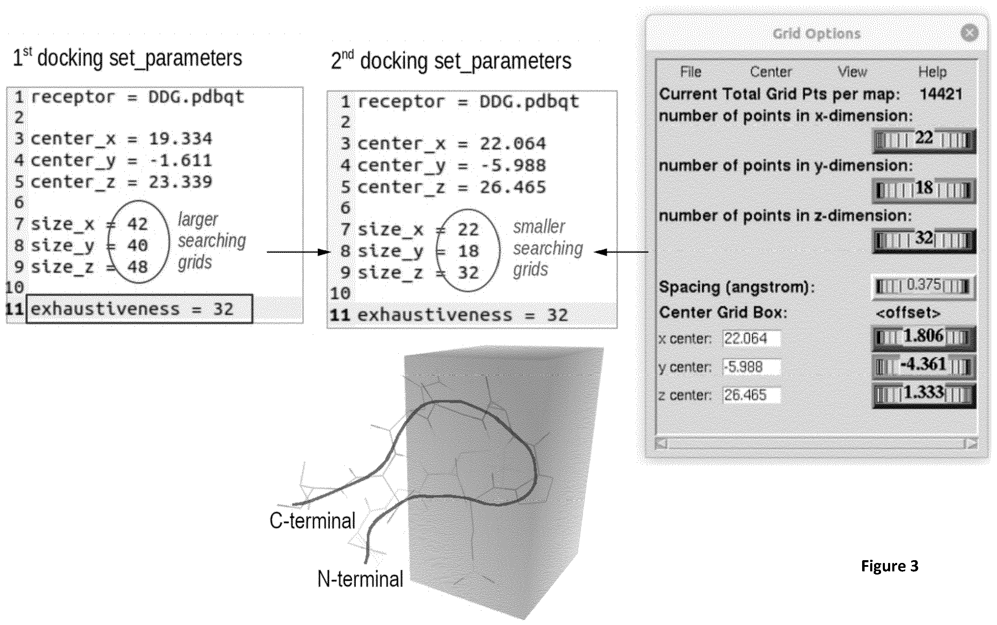

Figure 3 shows molecular docking parameters using AutoDock VINA (Trott, O., & Olson, A. J. AutoDock Vina: improving the speed and accuracy of docking with a new scoring function, efficient optimization, and multithreading. J. Comp. Chem. 2010, 31(2), 455-461. DOI: 10.1002%2Fjcc.21334.3) perl script.

A lower (more negative) docking score is indicative of a better binding affinity between the template and each type of functional monomer. The more negative the score, the stronger the predicted interaction. The most promising binding affinities and configurations have been extracted from both iterations (1st and 2nd docking sets), as follows: (i) AcA was chosen based on its -2.1 docking score, with its functional group oriented towards the inner core of the template, (ii) two NIPAM molecules were extracted having their docking scores -2.1 and - 2.0, respectively, (iii) six APMA molecules were initially extracted with scores ranging between -2.2 and -2.8, and (iv) one NPA molecule with binding score of -2.6 was selected for potential π-ring interactions with the proline amino acid, located in the middle of the template molecule sequence.

### 3. Molecular dynamics results for MIP design targeting DNAse-I

### 3.1 MIP recipe with 2 NIPAM vs 37 NIPAM molecules targeting DNAse-I

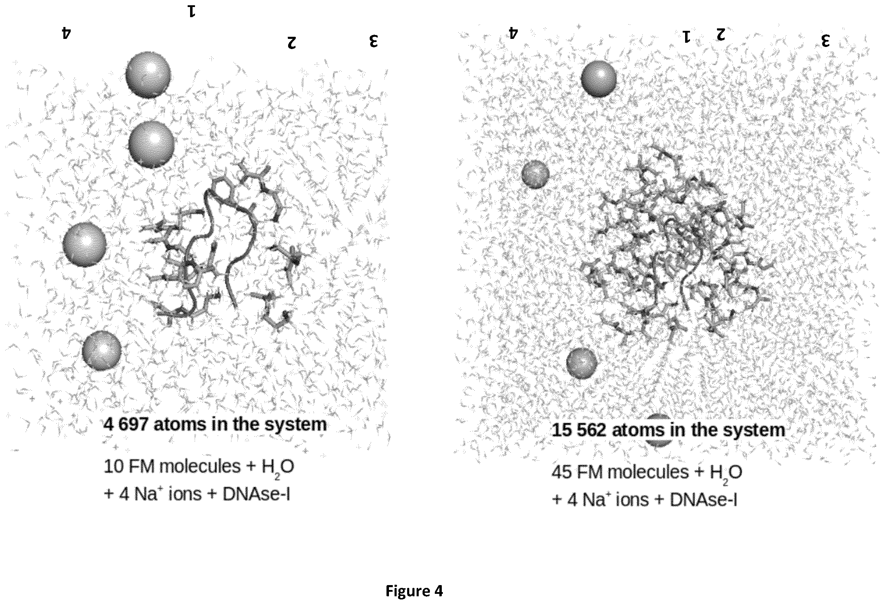

The docked (input) systems with 2 NIPAM (Figure 4, left) and 37 NIPAM (Figure 4, right) molecules 2 were solvated in SPC H2O model, neutralized with counter-ions, and subjected to multiple energy minimizations in 50000 steps. The minimized systems were then considered for short MD runs in NVT (300 K temperature), and NPT (1 bar pressure) ensembles. Finally, the equilibrated systems at desired parameters were subjected to 150 ns, in multiple replicas.

A MIP complexation system with 37 NIPAM was also designed, since NIPAM in higher concentrations exhibits a temperature-dependent phase transition, making it an attractive choice for creating MIPs with temperature-sensitive properties. Moreover, the temperature-dependent behavior of NIPAM-based MIPs provides the means to control the cargo release.

Here, the system with only 2 NIPAM molecules was considered as the control complex.

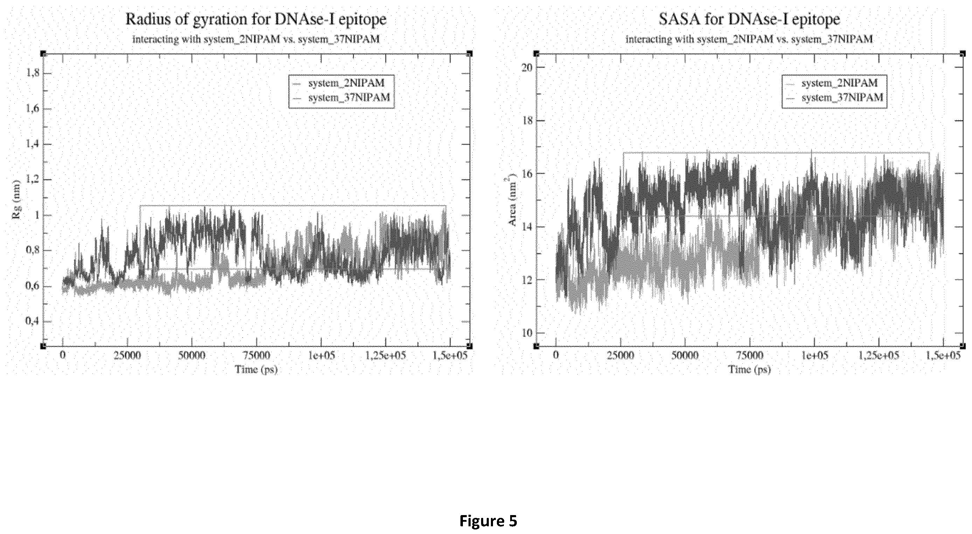

The structural (radius of gyration) and solvent-related behavior (solvent exposure area) of the selected template molecule (DDGCEPCGNDT peptide) in interaction with 2 NIPAM system vs. 37 NIPAM system was investigated. It was observed that, as expected, the presence of more NIPAM molecules in the system creates a higher degree of molecular crowding around the template molecule. This increased crowding affects the template's conformation and results in larger gyration values, prior to stabilization. With a larger number of NIPAM molecules, there may be increased opportunities for intermolecular potentials, such as van der Waals interactions or H-bonds, between the template molecule and NIPAM monomers. These interactions clearly influence the template's conformation and lead to larger gyration profiles (Figure 5, left).

The radius of gyration (Rg) profile of the template molecule was correlated with its solvent exposure area (Figure 5, right). The Rg of a molecule is related to its overall size and shape. In a more extended conformation (as seen for 37 NIPAM system), the template's surface area increases, which can impact solvent exposure.

At 300 K (26.85 °C), NIPAM is hydrophilic - below its lower critical solution temperature (LCST) of 32 °C - therefore it can easily form H-bonds with H2O molecules. Only above its LCST, NIPAM undergoes a phase transition, becoming hydrophobic, while the polymer chains collapse leading to the expulsion of H2O molecules. Moreover, the addition of NIPAM molecules also influences the dynamic behavior of the template molecule in a way that slightly increases its hydrophilicity, via conformational changes (Rg values) that expose different template's regions to the surrounding solvent.

After assessing the polymerization mixture's impact on the selected template, the focus is on hydrogen bonds' (H-bonds: 0.35 nm atomic distances) formation between the template and each/all functional monomers (see Table 1).

Table 1: Formed H-bonds number between the template molecule (T) and each functional monomer (FM) in the polymerization mixture during 150 ns MD productions at 300 K temperature - for both 2 NIPAM and 37 NIPAM systems. MIP - includes the analysis of all monomers together. Lowest values in bold.

**Table 1**

| DNase-FM | H-bonds number | |
|---|---|---|
| | 2 NIPAM system | 37 NIPAM system |
| T-AcA | **601** | **382** |
| T-APMA | 2102 | **1305** |
| T-NIPAM | **762** | 4426 |
| T-NPA | **443** | **176** |
| T-MIP | 3908 | 6289 |

H-bonds number between the template and all monomers is increased for systems with 37 NIPAM. However, a significant decrease in the number of formed H-bonds was observed between the template and AcA, APMA, and NPA when the system contains 37 NIPAM molecules, as compared to the system with only 2 NIPAM molecules in the mixture. Interestingly, it appears that in the system with more NIPAM molecules, there may be an increased competition for H-bonds with the template molecule. The NIPAM's crowding effects significantly affects the spatial arrangement and accessibility of the other monomers, making it more challenging for them to form H-bonds with the template.

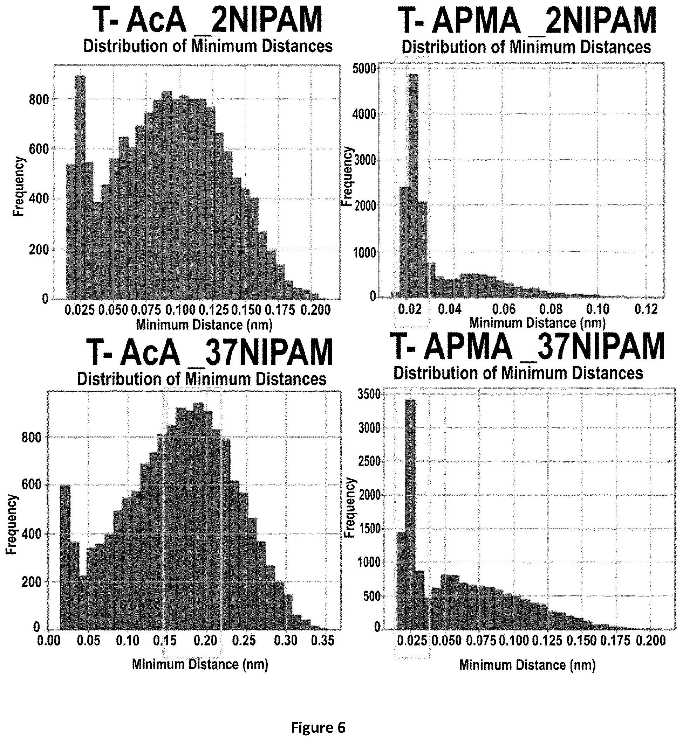

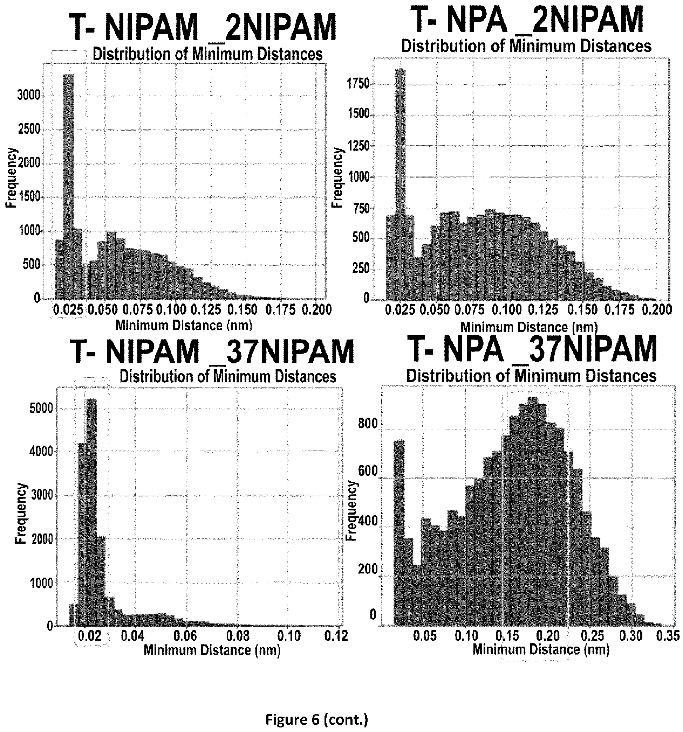

An essential aspect of the procedure also includes a comprehensive investigation of the minimum distances distribution (Figure 6) between the template molecule and each functional monomer.

The slightly narrowed distribution for systems with 37 NIPAM molecules, indicates more stable T-AcA interactions, when compared to the systems with lower NIPAM concentration. These interactions stabilize at distances around 0.16-0.18 nm. Between the template and APMA molecules, there are specific and robust interactions regardless of the number of NIPAM molecules in the system. The consistency in the narrowed distribution and peak positions between T-APMA in both systems suggests that the interactions maintain a stable binding mode.

Similarly, specific, and strong T-NIPAM interactions were obtained (0.025-0.125 nm) particularly with 37 NIPAM molecules in the system, for which even lower average minimum distances were observed. ForT-NPA complexes, the interactions are less robust and/or specific when less NIPAM molecules are present in the polymerization mixture. Consistently, the system with 37 NIPAM molecules exhibits a wider distribution of minimum distances between template and NPA, ranging from 0.05 to 0.3 nm. This distribution implies that T-NPA interactions are more variable and less specific.

It should be noted here that the minimum distances below 0.2 nm are correlated with covalent interactions between the template molecule and each functional monomer.

### 3.2 Cross-linker (BIS) concentration selection for MIPs targeting DNAse-I

Interestingly, as the amount of BIS molecules increases (from 10 BIS to 20 BIS to 40 BIS), there is a significant decrease in the number of H-bonds between the template molecule and AcA, APMA, and NPA monomers, however stabilizing the H-bonds between T-NIPAM. A stabilization between T- MIP+BIS for the 40 BIS system could clearly be identified. Hence, NIPAM together with BIS plays a crucial role in forming H-bonds with the template molecule, while stabilizing the polymerization matrix.

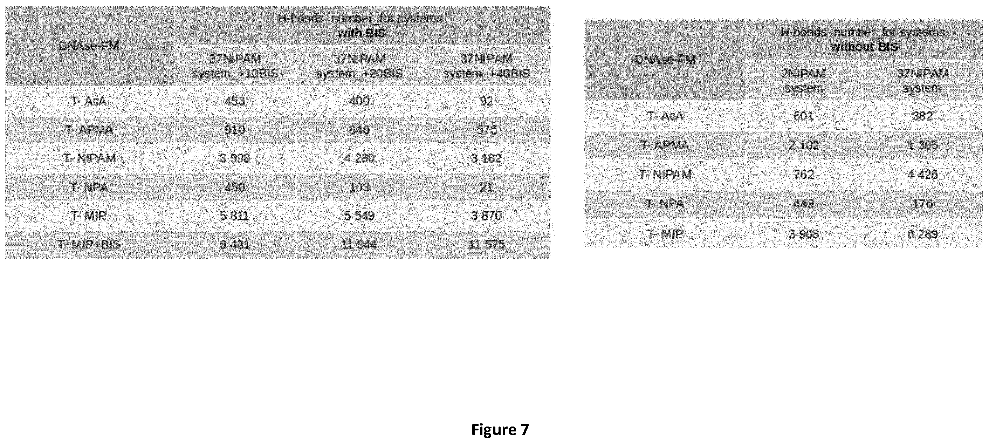

At least two factors explain why systems without BIS (Figure 7) but with high NIPAM concentrations form more H-bond counts: (i) BIS introduces steric hindrance, limiting FM-template interactions; (ii) high BIS concentration levels create dense networks that restrict FM and template mobility.

Importantly, by increasing the number of BIS molecules from 10 to 20 and then to 40, the average minimum distance between the functional monomers and BIS decreases. The average minimum distance remains the same (of 0.0195 nm) for both systems with 20 and 40 BIS molecules. This consistency clearly suggests that there is a saturation point or a limit to how close the BIS molecules can get to the functional monomers. This information is highly valuable for understanding the cross-linking behavior and its effects on template-monomer interactions.

The average minimum distances analysis suggests that the presence of BIS molecules in the complexation processes enhances and stabilizes the interactions between the template molecule and monomers, leading to slightly smaller minimum distances for systems with 20 BIS molecules. The minimum distance analysis is consistent with the obtained H-bonds profiles.

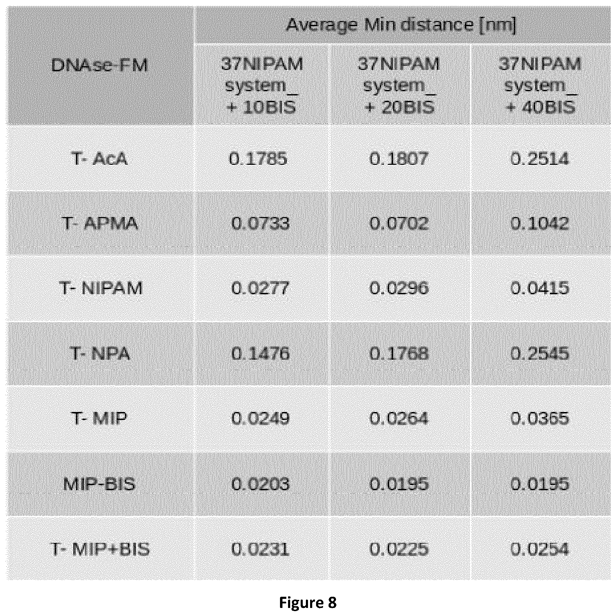

In summary, the behavior of functional monomers in MIP systems is influenced by the concentration of BIS (Figure 8). APMA maintains relatively strong correlations with neighboring components, indicating consistent interactions in systems with 10 and 20 BIS molecules. NIPAM's distance from other components also increases with BIS concentration, while NPA exhibits non-defined RDF behavior in the presence of 40 BIS. These findings suggest that BIS concentration plays a significant role in shaping the spatial arrangement and interactions of functional monomers in all three MIP systems.

### 3.3 Interaction energy investigation at different temperatures (280-310 K) for MIPs targeting DNAse-I

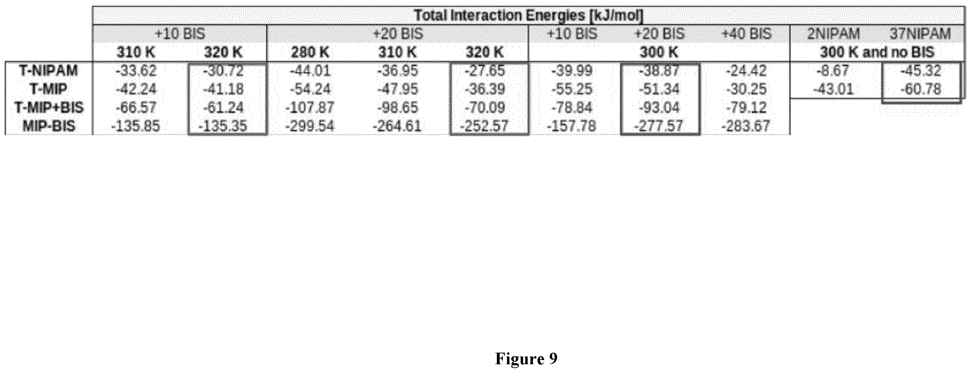

An analysis was performed based on total interaction energy calculations between the template molecule and NIPAM/all functional monomers (see Figure 9).

The presence of the cross-linker BIS enhances interactions within the systems, indicating its fundamental role in stabilizing the MIP structure. Clearly, the strength of interactions can be tuned by varying the concentration of the cross-linking agent (Figure 9), making it a critical factor in designing and optimizing the performance of the designed MIPs.

While BIS is important for the overall structure and stability of the MIP, the strength of specific interactions between template-monomers also depend on the complementarity of the binding sites, and the accessibility of functional monomers - that may be hindered by BIS, and the nature of involved functional groups.

The total interaction energy results (Figure 9) indicate that MIP system with 20 BIS molecules at 300 K demonstrate the strongest total interaction energies, making these conditions the most effective for stabilizing the template-monomer-crosslinker interactions. The optimal cross-linker concentration of 20 BIS creates a balance between structural rigidity and flexibility, allowing better accessibility of functional monomers to the template without over-restricting molecular mobility, as seen in denser systems with higher BIS levels (e.g., 40 BIS). At 300 K, the moderate thermal conditions further prevent the excessive weakening of non-covalent interactions, ensuring the template remains strongly bound within the polymer matrix. This combination of optimal cross-linking and temperature stability makes MIP system with 37 NIPAM and 20 BIS molecules at 300 K ideal for subsequent experimental synthesis and validation.

The observed decrease in total interaction energy between template-monomers with increasing temperature may be attributed to several factors. As temperature rises, increased molecular motion weakens non-covalent interactions like hydrogen bonds and van der Waals forces, while changes in solvent properties (e.g., density) affect solute-solvent interactions, impacting template-monomer kinetics. Higher temperatures also disrupt hydrophobic interactions due to increased solvent disorder and may induce conformational changes in the template, altering binding site accessibility and functional group orientation.

The above results can be summarized as follows:
- A suitable template molecule, DDGCEPCGNDT peptide, was extracted from the DNase-I structure using molecular dynamics methods.
- Extensive molecular docking studies were conducted to pinpoint the most suitable binding modes and conformations of potential monomers against the DDGCEPCGNDT peptide. The docked configurations are used as input coordinates for subsequent molecular dynamics investigations.
- An increase in the total number of H-bonds was observed between the template and all monomers in systems containing 37 NIPAM molecules.
- In higher concentration, NIPAM molecules may out-compete other monomers for H-bonding interactions with the template.
- The average minimum distance remained consistent for systems with 20 and 40 BIS molecules, suggesting a saturation point in BIS's interaction sites. The optimal cross-linker concentration was established at 20 BIS molecules.
- AcA and NPA were considered for exclusion from the polymerization mixture in further experimental implementations.
- The final MIP recipe comprises APMA, NIPAM (in highest concentration), and BIS at a moderate concentration of 20 molecules, with the most suitable complexation temperature of 300 K.

### II. Machine Learning (ML)-based MIP algorithm development

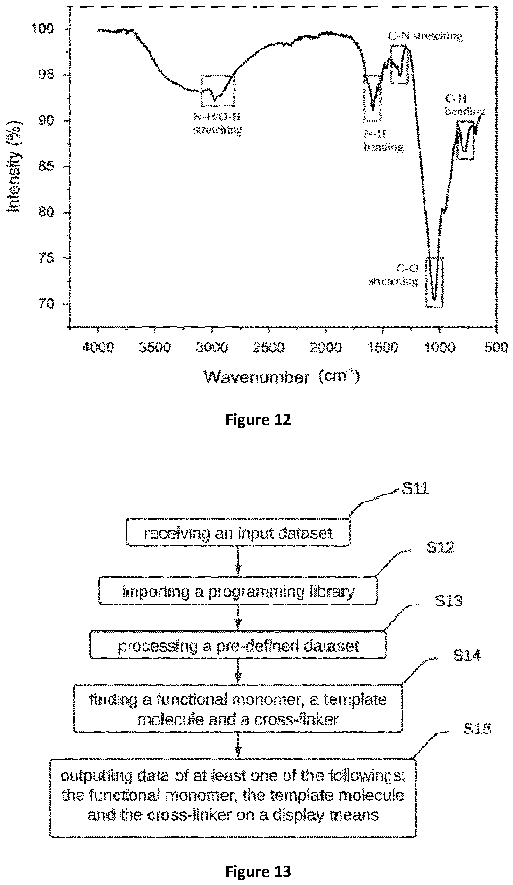

According to the embodiment of Figure 13, the computer-implemented method for producing a multifunctional molecularly imprinted polymer nanoparticle based on a machine learning algorithm comprises the following steps:
importing a programming library;
receiving an input dataset;
(S13) processing a pre-defined dataset, wherein the pre-defined dataset comprises data of functional monomers, template molecules and cross-linkers;
(S14) finding a best-performing functional monomer, and a cross-linker, wherein the functional monomer, the template molecule and the cross-linker have a maximum number of H-bonds, increased gyration values, minimum distances between MIP components, and a more negative value of total interaction energy based on the input dataset and selection criteria; and
(S15) outputting data of at least one of the followings: the functional monomer and the cross-linker specific for a given template molecule by user on a display means;
wherein the sequence of precedence of the step of receiving an input dataset and importing a programming library can be interchanged; and wherein in step (15) also output a ratio of the functional monomer and the cross-linker for the given template molecule (sequence) by user.

Firstly, the necessary libraries are imported, such as Pandas for data handling, scikit-learn for machine learning (ML).

We defined a function to predict best and optimal MIP components based on indicated selection criteria (in our case, maximum number of H-bonds, increased gyration values, minimum distances between MIP components, and a more negative value of total interaction energy, within a relaxed threshold). This function maps functional monomer types to their chemically complementary amino acid residue types, and is applied to each row in the dataframe, and the classification results are stored in a new column "Performance" automatically updated within the input dataset, for example, a CSV dataset.

It can be understood that the selection criterion of the "best functional monomer" of the present invention lies within the following four critical aspects.
(1) Correlated dependencies: the selection criterion of the present invention incorporates tightly correlated interdependencies between multiple physico-chemical metrics. As an example, H-bonds are considered together with minimum distances, and gyration values; while the total interaction energy is considered together with other metrics such as: minimum distances, gyration, H-bonds. None of the metrics used in the present invention is considered separate from the others. These metrics must all, together (concomitantly), satisfy the selection criteria condition.
(2) Contextual relevance: the correlated dependencies stated in point (1) are tightly tied to the specific context of the system being studied, nuances that are otherwise overlooked can thus be captured. For example, each of hundreds of different contexts gives totally different T-FM combinations - each with its unique physico-chemical behavior. This uniqueness comes from how each system is designed, the parameters used to simulate and synthesize, alongside the levels of randomness assigned for each T-FM interaction included in the dataset of the present invention.
(3) Unique metric ranges: the novelty/uniqueness of the selection criterion of the present invention is in particular the specific ranges of values these metrics exhibit when correlated. These ranges, for example, H-bonds 1000-4000 and interaction energies (-243 kJ/mol to -469 kJ/mol), derived solely from the training dataset of the present invention, are unique to the present invention.
(4) Weighted importance: the selection criterion of the present invention includes weighted labels that assign contextual importance to each metric, enabling predictions that depend heavily on these calibrated ranges (for example, H-bond counts between 1000-4000). This range, and its interrelation with other metrics, is both unprecedented and indispensable to our predictive process.Therefore, the selection criterion of the present invention is novel and inventive because it is multivariate, contextually aware and correlated. Furthermore, it uses weighted labels and fine-tuned ranges to meet the criteria for final predictions, and the direct link between metric calibration enhances predictive power and prediction accuracy.

In some embodiments, exception handling can be used to ensure that the code includes mechanisms to successfully handle errors or any unexpected issues that might occur during the algorithm's execution.

For training purposes, data splitting is used to split the data into training and testing sets is a fundamental practice to assess the performance of a trained model and make sure that it can perform well to novel/new, unseen data (such as new template molecules), and to avoid potential over-fitting behavior.

Our ML algorithm not only splits the data into training and testing sets to simulate "real-world scenarios", but also provides additional parameters. In the present exemplary embodiment, "test_size=0.2" means that 20% of the data is reserved for testing, while the remaining 80% is used for training the ML model. As the input dataset dimension increases, these parameters can be adjusted.

A "random_state" parameter is used in our ML algorithm, which ensures that the random splitting of the data into training and testing sets is reproducible. It basically performs as a random generator for data splitting. This parameter is useful for assessing reproducibility and comparing our model's performance across different runs/iterations.

The training dataset contains parameters for all MIP components: templates, monomers, and cross-linkers. In the polymerization process, these components are interdependent. When the ML output identifies a suitable monomer, concurrently identifies the crosslinking agent and the monomer-crosslinker ratios for a given template sequence prompted by users, best suited for developing/designing novel MIP recipes. This integration is rooted in the fact that the training dataset, derived from established and experimentally validated recipes, consistently includes templates, monomers, and cross-linkers.

The input dataset brings specific features for all MIP components. For instance, binding affinities reflect not only the monomers but also the template molecules (since templates and monomers interact with each other). On the other hand, template molecules also have their own properties integrated into the pre-defined dataset - such as sequence, secondary structure, gyration and solvent exposure data.

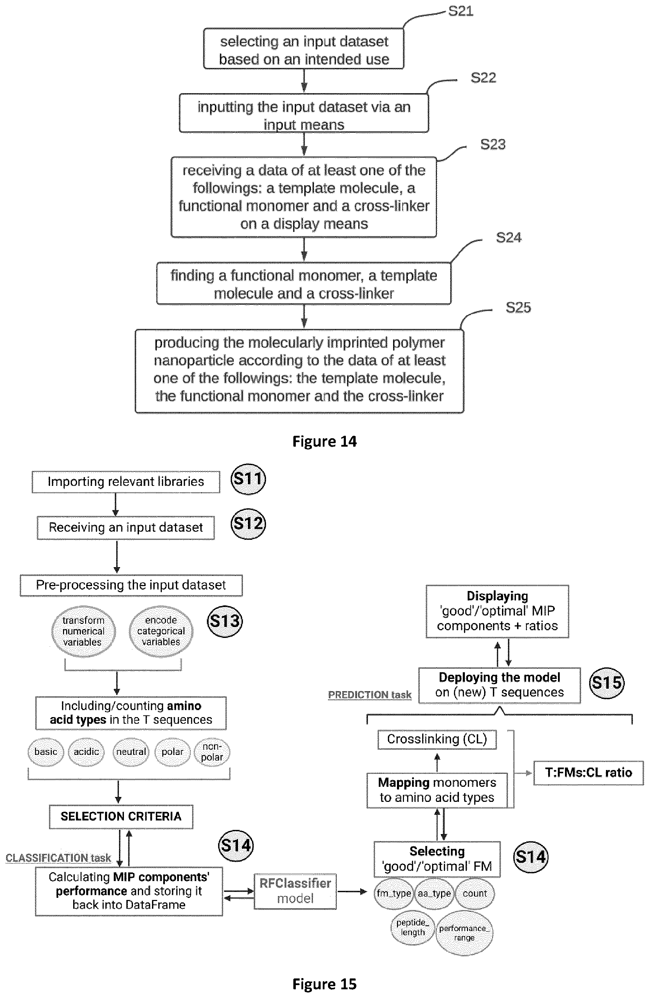

According to the embodiment of Figure 14, the method for producing a multifunctional molecularly imprinted polymer nanoparticle based on a machine learning algorithm, preferably the above ML algorithm, comprises the following steps:
(S21) selecting an input dataset based on an intended use;
(S22) inputting the input dataset via an input means;
(S23) receiving a data of at least one of the followings: a template molecule, a functional monomer, a cross-linker and a template-monomer-crosslinker ratio on a display means, wherein the data is obtained according to a computer-implemented method for producing a multifunctional molecularly imprinted polymer nanoparticle based on a machine learning algorithm; and
(S24) producing the molecularly imprinted polymer nanoparticle according to the data of at least one of the followings: the template molecule, the functional monomer and the cross-linker.

### III. Experimental section - MIP-NP synthesis for targeting DNAse-I

This example provides an overview of the experimental procedure considered to obtain all the experimental data points included in our input dataset.

### 1. Materials and reagents

Glass beads of 0.75-1.0 mm were purchased from Carl Roth (Karlsruhe, Germany).

The selected DNase-I peptide with DDGCEPCGNDT sequence (SEQ ID NO: 1) was synthesized by, and purchased from GenScript Biotech (Netherlands) BV.

The N'-tetramethylethylenediamine (TEMED), phosphate-buffered saline (PBS), N-5-isopropylacrylamide (NIPAM), N,N' methylenebisacrylamide (BIS), N-(3-aminopropyl) methacrylamide hydrochloride (APMA), and ammonium persulphate (APS), were purchased from Sigma Aldrich (Steinheim, Germany). Glutaraldehyde (GA), (3-Aminopropyl)triethoxysilane (APTES) and ethanolamine were purchased from Sigma Aldrich (Steinheim, Germany).

Double-distilled water, methanol, and ethanol were purchased from Carl Roth (Karlsruhe, Germany). Carl Roth also provided syringe filters Rotilabo PTFE (Karlsruhe, Germany). Sodium hydroxide (NaOH) was purchased from VWR (Dresden, Germany). Acryloxyethyl thiocarbamoyl Rhodamine B (RhB monomer) was obtained from Polysciences Europe GmbH (Hirschberg an der Bergstrasse, Germany). All chemicals and solvents were high-performance liquid chromatography (HPLC)/analytical grade and were used without any further purification. PBS solutions were prepared by dissolving each PBS tablet 200 mL of double-distilled water at room temperature (RT) to obtain a 0.01 M PBS buffer solution (pH 7.4) which was then filtered using syringe filters.

### 2. Instrumentation and experimental apparatus

During the synthesis process, a mechanical stirrer (IKA, Germany) was used for mixing purposes, a vortex (IKA, Germany), and a bath ultrasonicator (Eydam GmbH, Germany) was used for homogenous polymer growth and preventing agglomerations. A vacuum pump (Fischer Scientific GmbH, Germany) was employed for drying purposes.

Afterwards, the hydrodynamic size distribution and zeta potential (ZP) of resulting particles was characterized by dynamic light scattering (DLS) using Zetasizer Pro (Malvern Panalytical Ltd, United Kingdom) at different temperatures of 25 ± 0.1 °C, and 38 ± 0.1 °C, respectively.

The Cary 630 FTIR spectrometer was used in order to obtain Fourier transform infrared (FT-IR) in the range of 4000-650 cm⁻¹ (Agilent, USA).

### 3. Synthesis procedure of MIP-NPs

### 3.1 Functionalization of the solid support

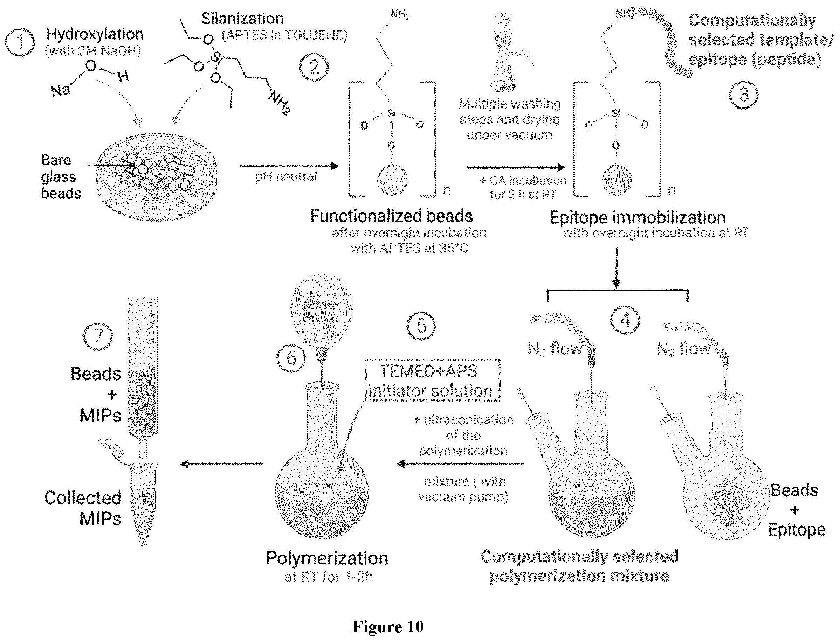

For synthesizing the computationally-derived MIP-NPs the solid-phase method was used. This method capitalizes on the principles of molecular imprinting, enabling the design and fabrication of highly selective and tailored nanoscale materials. In this approach (see Figure 10), glass beads serve as the solid support matrix, providing a scaffold for the MIP to form around template molecules, resulting in MIP-NPs with precisely defined recognition sites. This synthesis method offers several advantages, including ease of handling, scalability, and the potential for diverse applications.

In the first step of our synthesis, 60 g of bare glass beads were functionalized with 40 ml of 2M NaOH. This hydroxylation process alters the surface properties of glass beads, making their surface chemistry highly hydrophilic. Moreover, the immobilization of hydroxyl groups facilitates the binding of template molecules, and enhances the stability and compatibility of the bare glass beads with the polymerization process, while ensuring that the solid support remains intact during the synthesis.

The beads+NaOH were subjected to boiling on a heater for 15 min. Afterwards the pH of the solution was measured. Since the initial pH was too high (basic), additional washing steps with 50 ml DI H2O were considered, until reaching a final pH of 6.4. To accelerate the drying process, the functionalized beads were washed with 50 ml acetone in two rounds, then dried for 1h under vacuum.

A silanization step is necessary before the template binding interactions can take place. Silanization involves the attachment of silane compounds - that contain amino (NH2) groups - to the surface, which act as linkers or coupling agents. It prepares the surface for subsequent chemical modifications and ensures proper binding sites for the template molecules. These NH groups serve as binding sites for template molecules through hydrogen bonding or covalent bonding, depending on the nature of the template and the functional groups present. The silanization solution was prepared as follows: 47 ml toluene + 1 ml APTES, without N2 flow. A higher volume was used to make sure that APTES solution properly covers the beads. At this stage, the beads were incubated overnight (ON) at 35°C in water bath.

### 3.2 DNase-I peptide immobilization on solid support

Since toluene is not miscible in H2O, after ON incubation, the beads were washed 4 times with 50 ml/each acetone, and 2 times with 50 ml/each methanol. Then the beads were dried for 1 h under vacuum.

For covalent bindings enhancement, GA incubation was used. It stabilizes covalent attachments through NH2, significantly important for anchoring template molecules during the molecular imprinting process. Importantly, the concentration and reaction time of GA can be adjusted to control the density of binding sites on the solid support. For GA incubation 13.44 ml GA in 34.56 ml PBS were used. The incubation took place at RT for 2h.

Except from making the solution more organic, the use of GA in PBS helps to stabilize the functionalized glass beads surface. This stability is essential for long-term applications where the functionalized beads need to maintain their binding capacity over extended periods.

Prior to peptide immobilization, the beads were again washed multiple times with 50 ml DI H2O, and dried for 20 min under vacuum.

Subsequently, 10 mg of the peptide selected in section 1 (sequence: DDGCEPCGNDT), dissolved in 40 ml PBS, was added and incubated overnight with the beads at RT.

### 3.3 MIP-NP synthesis

In the following stage of the synthesis procedure, after discarding PBS and washing beads - this time via pipetting, the beads that had been functionalized with the template molecule were immersed in a sodium borohydride (NaBH4) solution for Schiff base reduction, a process lasting 30 minutes. After binding of template molecule, the reduction step transforms the Schiff bases into enduring secondary amines. This transformation firmly establishes the template binding sites, fortifying their resilience and precision, thereby amplifying the effectiveness of the imprinting process.

The NaBH4 solution consisted of 35 mg of NaBH4 in 35 ml PBS, with which beads were incubated for 30 min at RT.

Afterwards we incubated the beads with 50 ml/10mM blocking agent solution (49.5 ml PBS + 500µl ethanolamine) for 15 min. Considering that ethanolamine-treated surfaces are less prone to non-specific bindings, ethanolamine was also used to quench any un-reacted aldehyde groups on the solid support. It reacts with any remaining aldehydes, converting them to stable secondary amines. This step ensures that all available aldehyde sites are blocked, preventing unwanted/non-specific interactions during the imprinting process. Additionally, ethanolamine passivation helps maintain the integrity and stability of the imprinting sites, contributing to the long-term performance of our MIPs.

The beads were then washed multiple times (5x) with DI H2O and dried under vacuum for 1 h.

Meanwhile, the following polymerization solution in 100 ml total volume was prepared:
˙8.78 mg APMA - dissolved in 1 ml milliQ H2O;
˙34.09 mg NIPAM - dissolved in 1 ml milliQ H2O;
˙25.10 mg BIS - dissolved in 1 ml milliQ H2O;
˙2 mg RhD - dissolved in 1 ml ethanol.

An additional 1 ml solution was prepared for initiating the polymerization process of the monomers mentioned above. The initiator solution consists of: (a) 1.09 mg APS in 5 ml milliQ H2O, and (b) 3 µl TEMED in 497µl milliQ H2O. Then 500 µl of solution (a) was mixed with (b).

The rest of 95 ml (out of 100 ml total volume) of milliQ H2O was poured in a round-bottom flask.

The dried beads were transferred, using a glass funnel, into a 250 ml round-bottom flask and subjected to N2 flow for 20 min. Concomitantly, the polymerization mixture underwent ultrasonication under vacuum for 20 min, followed by another 30 min of bubbling.

To prevent the entry of O2 molecules into the polymerization mixture and the functionalized beads, two N2-filled balloons were employed to transfer the polymerization mixture onto the beads. Finally, the initiator solution was added into the flask with the functionalized beads and polymerization mixture. The polymerization took place at approximately 300 K temperature for 2h.

By employing a filtration column and vacuum pump, the initial supernatant was eliminated, which now solely consisted of unbounded components. Subsequently, the beads were washed in 3 rounds with 20 ml of cold (7.8°C) milliQ H2O.

Finally, the MIPs were collected by employing 3 rounds of 20 ml hot wash (68°C), with 1 min interval between each round.

### 4. Results and discussion

As already mentioned in the section 3.1 above, poly-NIPAM is a well-known thermo-responsive polymer that undergoes a phase transition in response to changes of temperature. At temperatures below its lower critical temperature (LCST) (around 32°C), poly-NIPAM is hydrophilic, therefore highly soluble in H2O. However, as the temperature exceeds its LCST, it undergoes a phase transition and becomes hydrophobic (Tsung-Yu Wu, Alyssa B. Zrimsek, Sergei V. Bykov, Ryan S. Jakubek, and Sanford A. Asher. Hydrophobic Collapse Initiates the Poly(N-isopropylacrylamide) Volume Phase Transition Reaction Coordinate. J. Phys. Chem. B 2018, 122 (11), 3008-3014. DOI: 10.1021/acs.jpcb.8b00740.4.

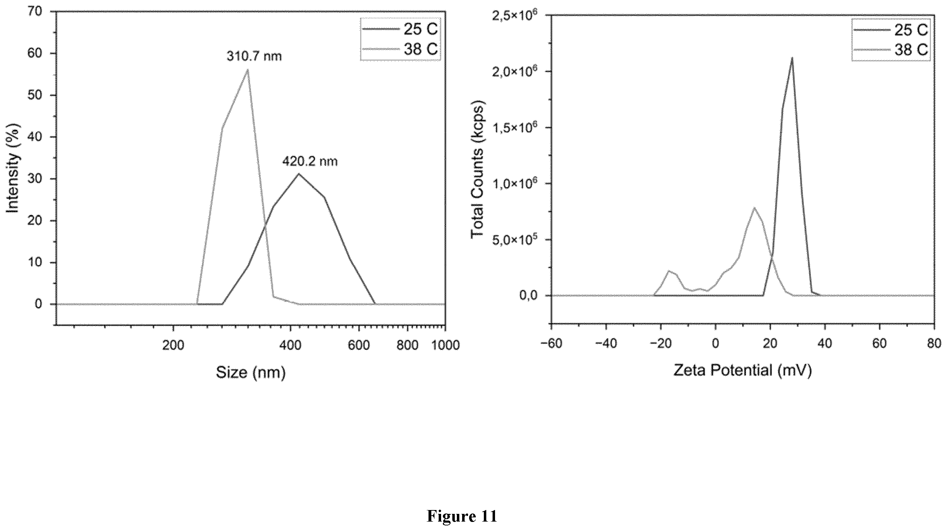

In this context, the thermo-responsive profile of our MIP-NP with a high concentration of NIPAM was experimentally addressed (Figure 11, left).

At 25°C the temperature is below the LCST of poly-NIPAM. The polymer remains in a hydrophilic state, and the MIP-NPs are well-dispersed in the solution, resulting in larger particle sizes (of 420.2 nm). In contrast, at 38°C, the temperature exceeds the LCST of poly-NIPAM. As a result, poly-NIPAM becomes hydrophobic, leading to MIP-NPs smaller in size (310.7 nm). This behavior proves the thermo-responsive capability of the synthesized MIP-NP.

The zeta potential (ZP), also known as the electrokinetic potential, is the electrical potential at the surface of tiny particles in a colloid (a mixture where tiny particles are dispersed in a liquid) when they move under the influence of an electric field. It measures how much work is required to bring a single positive charge from far away to the particle's surface without speeding it up. It provides information about the difference in potential between the electric double layer around the moving particles and the layer of liquid surrounding them at the surface (Sourav Bhattacharjee. DLS and zeta potential - What they are and what they are not? JCR 2016, 235, 337¬351. DOI: 10.1016/j.jconrel.2016.06.0175).

Since the temperature variation influences the size distribution of the MIP-NPs, the ZP changes from 25°C to 38°C as well. Figure 11 (right) shows the poly-NIPAM response, as the temperature increases, from higher (at 25°C) to lower (at 38°C) stability.

The interpretation of Fourier-Transform InfraRed (FT-IR) spectra involves analyzing the positions of absorption peaks (wavenumbers) and their corresponding functional groups and vibrational modes.

The C-H bending modes from 800 cm⁻¹ is indicative of hydrocarbons. The deep and strong C-O band at 1000 cm⁻¹ outlines stretching of carbonyl or ether groups. Typically, between 1000 cm⁻¹ and 1350 cm⁻¹, C-N stretching modes are associated with amines, amides, and/or other nitrogen-containing groups.

N-H bending modes found at ^{~}1500 cm⁻¹ confirm the presence of amine or amide functional groups in the polymer.

A weak band at 1300 cm⁻¹ for O-H bending modes associated with the presence of hydroxyl (alcohol) groups could also be identified.

Finally, the most extended range, from 2300 cm⁻¹ onward - corresponds to O-H stretching for intermolecular bonding, and N-H stretching from primary amino groups.

Importantly, the characteristic vibrational modes of NIPAM monomer at around 2800-3000 cm⁻¹ for C-H stretching, C-N stretching at 1230-1250 cm⁻¹, and N-H bending at around 1550-1600 cm⁻¹ have been identified.

### Example 2

### I. Materials and methods

### 1. Molecular docking and MD methodology considered for all computational data points in the input dataset

All functional monomers (FMs) were modeled using Chimera software (Pettersen, E.F., Goddard, T.D., Huang, C.C., Couch, G.S., Greenblatt, D.M., Meng, E.C., & Ferrin, T.E. (2004). UCSF Chimera-A visualization system for exploratory research and analysis. Journal of Computational Chemistry, 25(13), 1605-1612. DOI:10.1002/jcc.20084), by fetching each FM's ID from PubChem database (Kim, S., Chen, J., Cheng, T., Gindulyte, A., He, J., He, S., & Bolton, E.E. (2019). PubChem 2019 update: improved access to chemical data. Nucleic Acids Research, 47(D1), D1102-D1109. DOI: 10.1093/nar/gky1033). In terms of template (T) molecules, prior and extensive MD productions (detailed below) were carried out to extract the best epitopes from whole target molecules, for almost all of the investigated MIP recipes within the dataset. The software used for the visualization and refinement purposes was PyMol (Schrödinger, L.; & DeLano, W. (2020). PyMOL. Retrieved from http://www.pymol.org/pymol). Input coordinate files for all target molecules were retrieved from Protein Data Bank database (Berman, H. M., Westbrook, J., Feng, Z., Gilliland, G., Bhat, T. N., Weissig, H., ... & Bourne, P. E. (2000). The Protein Data Bank. Nucleic Acids Research, 28(1), 235-242. DOI: 10.1093/nar/28.1.235 ). For FMs modeling - particularly for the addition of hydrogen atoms and initial structure optimizations using Universal Force Field (UFF) (Anthopoulos, A., & Cameron, !. (2022). Optimized GPU implementation of Merck Molecular Force Field and Universal Force Field. Journal of Computational Chemistry, 43(1), 23-30. DOI: 10.1002/jcc.26829), the Avogadro software (Hanwell, M. D., Curtis, D. E., Lonie, D. C., Vandermeersch, T., Zurek, E., & Hutchison, G. R. (2012). Avogadro: An advanced semantic chemical editor, visualization, and analysis platform. Journal of Cheminformatics, 4(1), 17. DOI: 10.1186/1758-2946-4-17 ) was used. Owning a robust parameterization for a broad spectrum of molecular structures, UFF includes well-documented parameters for atom bonds stretching, angles bending, torsional angles, and non-bonded interactions.

Molecular docking studies were carried out using either HADDOCK server (G.C.P van Zundert, J.P.G.L.M. Rodrigues, M. Trellet, C. Schmitz, P.L. Kastritis, E. Karaca, A.S.J. Melquiond, M. van Dijk, S.J. de Vries and A.M.J.J. Bonvin. The HADDOCK2.2 webserver: User-friendly integrative modeling of biomolecular complexes. J. Mol. Biol., 428, 720-725 (2016)) - in vacuum, and/or AutoDock Vina software (Trott, O., & Olson, A. J. (2010). AutoDock Vina: improving the speed and accuracy of docking with a new scoring function, efficient optimization, and multithreading. Journal of Computational Chemistry, 31(2), 455-461. DOI: 10.1002/jcc.21334). The results obtained from the server targeted the best binding poses, having the highest (most negative) binding energies and docking scores, selected from the largest cluster sizes with the most negative Z-scores. With respect to AutoDock Vina set-up, Autodock Tools (ADT) (Morris, G. M., Huey, R., Lindstrom, W., Sanner, M. F., Belew, R. K., Goodsell, D. S., & Olson, A. J. (2009). AutoDock4 and AutoDockTools4: Automated docking with selective receptor flexibility. Journal of Computational Chemistry, 30(16), 2785-2791. DOI: 10.1002/jcc.21256) were used to set the polar hydrogen atoms for all FMs, and both polar and non-polar ones for each T molecule. For conformational space search, grid cells with different vector sizes were used; depending on the length of each T sequence of interest. The Gasteiger partial charges on all atoms were also assigned using the ADT. The output T-FMs docked complexes using AutoDock Vina were analyzed using Chimera. The best conformers selection was based on highest binding affinities, low RMSD values and highest number of H-bonds formed between each T molecule and each FM.

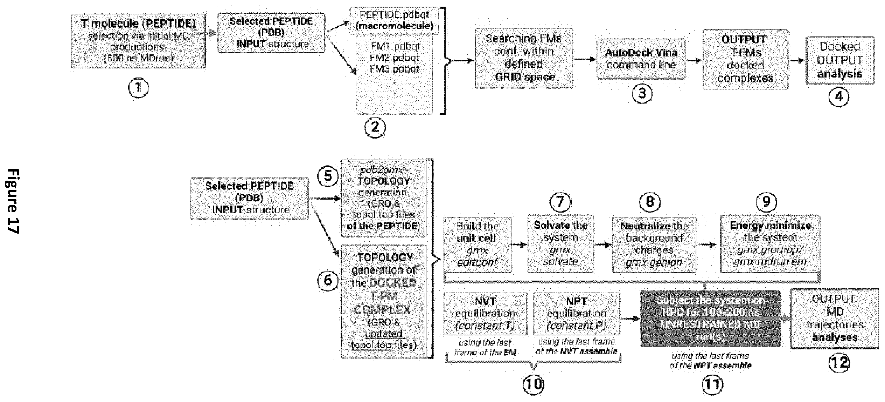

After each FM was modeled, since monomers are non-protein structures, parameterization steps using external force fields, i.e. CGenFF (Vanommeslaeghe, K., Hatcher, E., Acharya, C., Kundu, S., Zhong, S., Shim, J., & Mackerell, A. D. (2010). CHARMM general force field: A force field for drug-like molecules compatible with the CHARMM all-atom additive biological force fields. Journal of Computational Chemistry, 31(4), 671-690. DOI: 10.1002/jcc.21367) to obtain the monomers' parameter files and topologies were considered. Afterwards, all docked systems were subjected to extensive MD simulations using GROMACS software (GROMACS development team. "GROMACS 2023." GROMACS User Manual. (2023). Available at: https://manual.gromacs.org/documentation/2023/ [Accessed July 6, 2024]. DOI: 10.5281/zenodo.11104849), in multiple replicas. Figure 17 depicts the overall computational approach considered to extract template-monomer-crosslinker feature datapoints. CHARMM36 (Huang, J., Rauscher, S., Nawrocki, G., Ran, T., Feig, M., de Groot, B. L., Grubmüller, H., & MacKerell, A. D. Jr. (2017). CHARMM36: an improved force field for folded and intrinsically disordered proteins. Nature Methods, 14(1), 71-73. DOI: 10.1038/nmeth.4067.) force field (highly compatible with CGenFF) was used for all our MDs. The leapfrog integrator (Hockney, R. W., & Eastwood, J. W. (1988). Computer Simulation Using Particles. Taylor & Francis) was the numerical algorithm used for the all productions. This algorithm integrates Newton's equations of motion, and updates atom positions and velocities in a staggered manner. Moreover, leap-frog conserves energies better over long MD productions, when compared to other integrators, and it is efficient - while providing a good balance between accuracy and computational cost. Energy outputs and log file updates were configured to be saved every 100.0 ps, and the coordinates were similarly saved at this interval to monitor the T-FM systems dynamic evolution. The modeled systems were then solvated with TIP3P water model, neutralized with counter-ions, and energy minimized (multiple times until reaching their global minima) through the Steepest Descent algorithm (E. J. Haug, J. S. Arora, and K. Matsui. 1976. A steepest-descent method for optimization of mechanical systems. J. Optim. Theory Appl. 19, 3 (July 1976), 401-424. DOI: 10.1007/BF00941484) for 50,000 steps. Upon minimizations, the systems underwent NVT (constant number of particles, volume and temperature) and NPT (constant number of particles, pressure, and temperature) equilibration at temperature ranges of 280-310 K and 1 bar pressure for 10 ns/each/system. Moreover, temperature couplings were managed using the V-rescale thermostat (Bussi G, Donadio D, Parrinello M. Canonical sampling through velocity rescaling. J Chem Phys. 2007 Jan 7;126(1):014101. DOI: 10.1063/1.2408420), with separate coupling groups for the T-FM complexes and solvent - with or without ions, each maintained at the reference temperatures with a time constant of 0.1 ps. Pressure couplings were achieved via Parrinello-Rahman algorithm (Parrinello, M., & Rahman, A. (1980). Crystal structure and pair potentials: A molecular-dynamics study. Physical Review Letters, 45(14), 1196-1199. DOI: 10.1103/PhysRevLett.45.1196), enforcing isotropic scaling of the simulation box with a compressibility of 4.5e-5 bar-1.

All free dynamics simulations continued from previously equilibrated NPT ensembles, employing the LINCS algorithm (Hess, B., Bekker, H., Berendsen, H. J. C., & Fraaije, J. G. E. M. (1997). LINCS: A linear constraint solver for molecular simulations. Journal of Computational Chemistry, 18(12), 1463-1472. DOI: 10.1002/jcc.540181209) to maintain holonomic constraints, specifically on hydrogen bonds. The need for LINCS-based constraints emerges from the fast vibrational motions of hydrogen atoms (having low masses) - however being critical for the physicochemical properties of T molecules (e.g. folding behavior, stability, and interactions with FMs). By constraining these bonds, it was possible to use larger time steps, which significantly improved computational efficiency without sacrificing accuracy.

Additionally, neighbor searching was conducted using the Verlet cutoff scheme (Verlet, L. (1967). Computer "Experiments" on Classical Fluids. I. Thermodynamical Properties of Lennard-Jones Molecules. Physical Review, 159(1), 98-103. DOI: 10.1103/PhysRev.159.98) with a grid-based searching. The short-range van der Waals interactions were calculated using a force-switch modifier, with a cutoff range set between 1.0-1.2 nm. Long-range electrostatics were handled using the Particle Mesh Ewald (PME) method (Essmann, U., Perera, L., Berkowitz, M. L., Darden, T., Lee, H., & Pedersen, L. G. (1995). A smooth particle mesh Ewald method. The Journal of Chemical Physics, 103(19), 8577-8593. DOI: 10.1063/1.470117), ensuring the accurate treatment with a cubic interpolation order and a grid spacing of 0.16 nm for the Fast Fourier Transform (FFT).

Periodic boundary conditions (PBC) (Frenkel, D., & Smit, B. (2002). Understanding Molecular Simulation: From Algorithms to Applications. Academic Press) were applied in all three dimensions (xyz) to replicate the infinite system environment in all mdrun productions (energy minimization, NVT, NPT and free MDs). Dispersion corrections were not considered, as they are typically omitted for protein-based structures using the CHARMM36 [Huang J, MacKerell AD Jr. CHARMM36 all-atom additive protein force field: validation based on comparison to NMR data. J Comput Chem. 2013 Sep 30;34(25):2135-45. doi: 10.1002/jcc.23354.] additive force field. Initial velocities were not generated, assuming the continuation from the NPT equilibrated states of our T-FMs systems. The free, unrestrained dynamics were considered for 100-200 ns time-frames.

Finally, analyses of all trajectories per system were carried out using the Gromacs package (GROMACS development team. "GROMACS 2023." GROMACS User Manual. (2023). Available at: https://manual.gromacs.org/documentation/2023/ [Accessed July 6, 2024]. DOI: 10.5281/zenodo.11104849), Grace plotting tool (Grace Development Team. (2023). Grace - 2D plotting tool. Version 5.1.25. http://plasma-gate.weizmann.ac.il/Grace/), and visualization packages such as PyMol (Schrödinger, L.; & DeLano, W. (2020). PyMOL. Retrieved from http://www.pymol.org/pymol) and VMD (Humphrey, W., Dalke, A., & Schulten, K. (1996). VMD: Visual molecular dynamics. Journal of Molecular Graphics, 14(1), 33-38. DOI: 10.1016/0263-7855(96)00018-5), supplemented by Python scripts for further analyses. The key physicochemical quantities included:
solvent exposure and gyration profiles of T molecules
˙gyration profiles of T-FM formed complexes
˙total number of H-bonds between each T-FM pair
˙T-FM minimum distances
˙total interaction energies (the Σ between Lennard-Jones and Coulomb potentials).

### 2. ML algorithm development

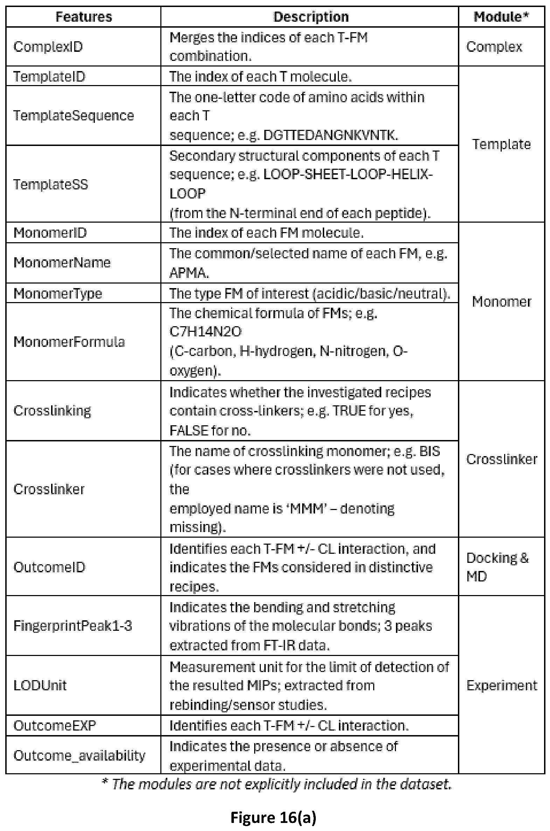

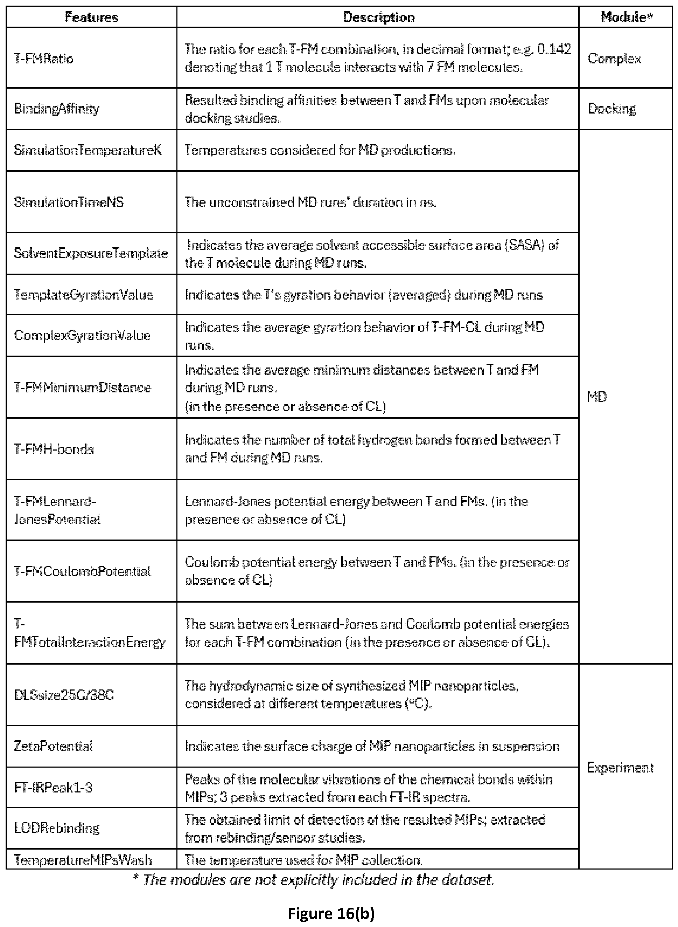

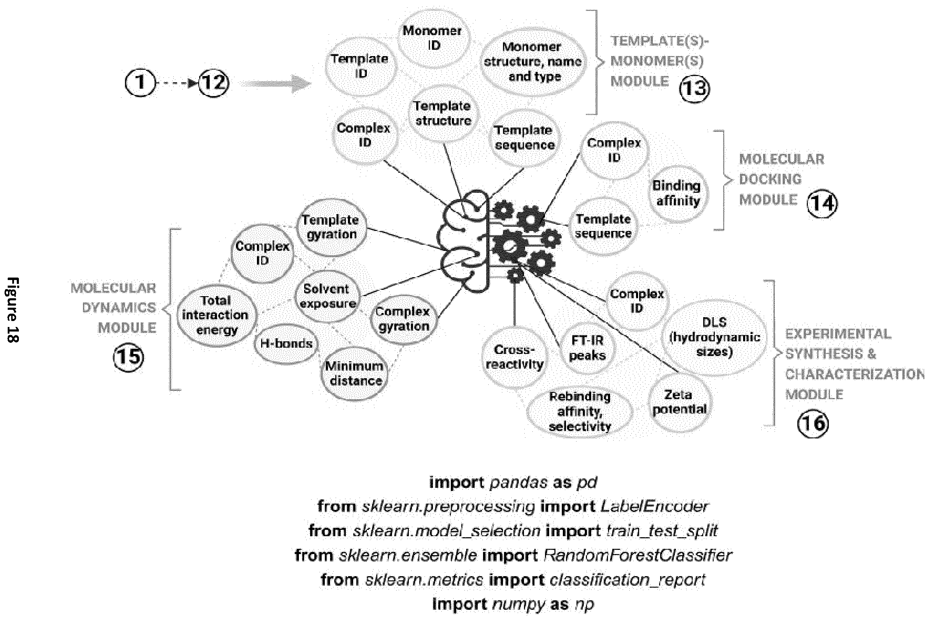

As an example, Python 3.8.10 version was used for scripting. The model's performance was assessed using metrics such as accuracy, precision, recall, and F1-score. Visualization tools like Matplotlib and Seaborn were used to illustrate the data distribution and model performance. The dataset was split into training and testing sets using 70/30 ratio. The input features used for our ML algorithm development are illustrated in Figure 18. More explicitly, the input features are listed in Figure 16(a) and 16(b).

The algorithm starts by loading the input dataset, and then converting all the numerical values to string, replaces commas with dots, and makes conversions to float. The categorical variables were encoded using *LabelEncoder* imported from scikit-learn's preprocessing module. The function used to count different types of amino acids (acidic, basic, neutral, polar and non-polar) within template sequences also included the special cases such as glycine, proline and cysteine. We considered glycine as non-polar amino acid in our function, although due to its small size, the lack of distinct hydrophobic side chain makes it rather unique. Similarly, proline forms a cyclic structure while its side chain is basically double-connected to the main chain (forming a secondary amine), and its hydrophobic ring structure fits well into the non-polar category. Cysteine in considered a special case due to its ability to form covalent disulfide bonds via the thiol (-SH) group in its side chain, and it is often classified as a polar one. All amino acid sequences followed the same format as strings in uppercase, while the algorithm also ensures that there are no additional spaces and/or characters before and after the actual strings. Afterwards, using pandas library, the algorithm prepares the *TemplateSequence* column in the dataframe, counts specific types of amino acids in each sequence, and stores these counts in new columns in the dataframe under basic_count, acidic_count, etc. format.

The selection criteria function for calculating predicted MIP components' performances directly uses the previously described amino acid features. This function evaluates if the conditions based on several features from dataframe and returns a classification (e.g. 'Good') if the criteria are met. One of the conditions checks if e.g. either acidic_count is greater than 0 or polar_count is greater than 0, meaning that the sequence must have at least one acidic or polar residue. Other conditions include specific numerical ranges of gyration values, minimum distances, H-bonds, and total interaction energies for all potential classes: 'good', 'bad' and 'optimal' MIP components. Importantly, all these conditions are combined using the and operator, which means every single condition must be true for the entire expression to be true. Herein, the structural characteristics of template molecules and their biochemical properties, alongside their involvement in dynamic interactions with distinctive monomers are combined. The 'e I if' (*else if*) conditional statements were used to sequentially check multiple expressions. Following the selection criteria, the algorithm calculates the performance of each T sequence in combination with multiple types of monomers, as per the obtained and integrated data in our input.

The MIP components' performance predictions are done by the tuned RFClassifier model. The workflow begins by splitting our dataset into training and testing sets using the train_test_split function. This function divides the feature matrix X and the target labels y_encoded into four groups: X_train, X_test, y_train, and y_test, with 70% of the data allocated for training and 30% for testing, ensuring reproducibility within a fixed random state seed (random_state=135). The RFClassifier is initialized with 400 trees and a maximum depth of 10, according to our tuned and tested hyperparameters. Using model.fit(X_train, y_train) the model learns patterns (training process) from the training dataset, while adjusting its parameters accordingly. Finally, the trained model is used to make predictions on the test data (X_test), predicting labels (y_pred) which are afterwards compared to the actual test labels, in order to evaluate the predictions' performance. These predictions are ultimately stored in the dataframe (under a separate column; and converted into categorical data type), and are subsequently used as input data for predictions on new, unseen template sequences.

The selection of best MIP components on unseen T sequences depends on several factors including the type of FM ('acidic', 'basic', or 'neutral'), the amino acid type within prompted T sequences, the amino acid type's count, peptide length, and the considered performance range (set as 0.5). We used the latter parameter to induce a flexible threshold for the most relevant feature, the total interaction energy between each T and FM in the presence/absence of crosslinking. The mathematical framework of this threshold is e.g. min_energy + 0.5 * |min_energy|. The absolute value of the most negative interaction energy in the second term correlates with optimal thresholds regardless if the energy is negative or positive. Moreover, clearly by adding a negative second term will significantly increase (in terms of negativity) the energy threshold, posing the risk of excluding too many MIP components that can still represent good candidates for best MIP formulations. Upon filtering the dataframe with the existing prediction performances, the algorithm samples randomly and selects one 'good' and one 'optimal' FM, if available. Another function was used to map monomers to their respective amino acid types by selecting the best FMs for acidic, basic, and neutral types and calculating their ratios according to each prompted (new) template sequence.

For crosslinking, the crosslinker ratio is established by filtering the dataframe to identify crosslinking (TRUE or FALSE), then converting their corresponding interaction energy terms to numeric format, and selecting the crosslinker with the most negative interaction energy value. So far, in our dataset the best crosslinking effects were observed for medium crosslinking concentrations (e.g. 5 molecules, or 1:5=0.2 in decimal format). Extreme crosslinking effects correlated in our MD productions with hindered template-monomer interactions, by causing particular monomer-crosslinker clusterization behavior.

Finally, besides the best performing MIP components and potential alternatives (classified as 'optimal'), the users receive as output a classification report as part of sklearn.metrics module in the scikit-learn library. This report provides the true labels for the test dataset (y_test), and the predicted labels (y_predict), and prints out the precision, recall, F1-score, and support metrics for each identified class ('good', 'optimal', 'bad' MIP components), providing a comprehensive evaluation of the RFClassifier model's performance - the same model that generalizes on new template sequences.

### II. Experimental validation of the ML-based MIP-NP formulations

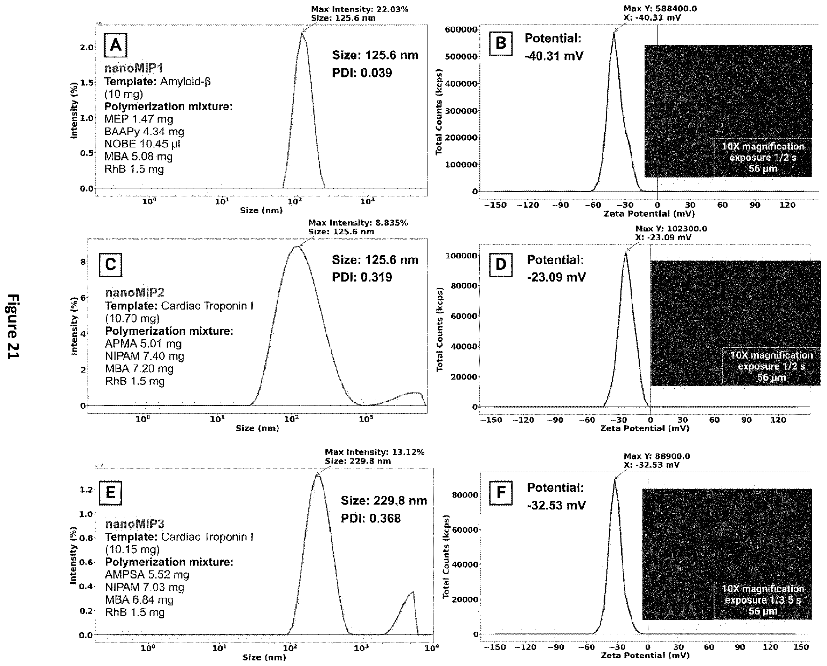

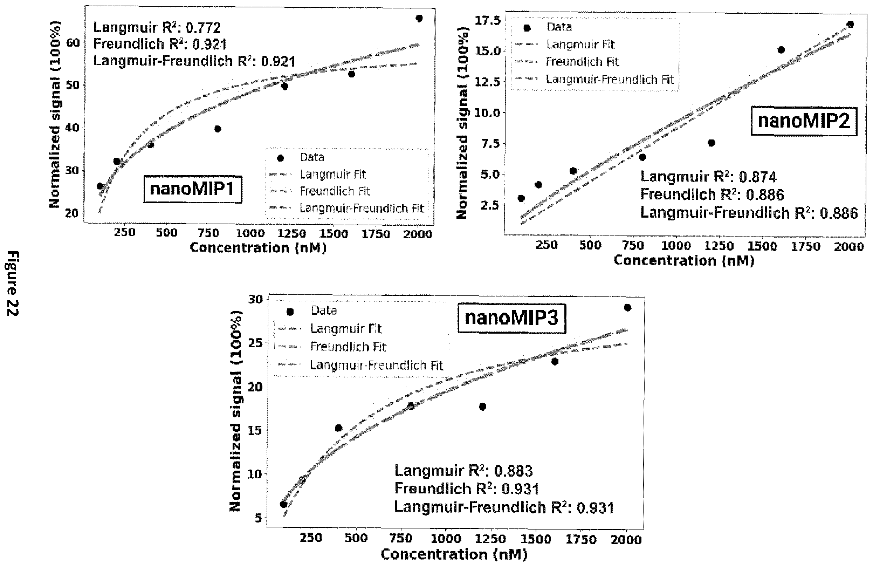

As an example of the present invention, the following shows the experimental studies part. The algorithm's performance was tested on two, unseen template sequences (Figure 20), amyloid-β (YEVHHQKLVFFA; SEQ ID NO: 3; PDB: 1IYT) and cardiac troponin-I (ISASRKLQLK; SEQ ID NO: 4; PDB: 4Y99) , achieving classification and prediction accuracy of 93% and 95%, respectively, based on predefined selection criteria with weighted features. Our algorithm identified three promising formulations - one for amyloid-β and two for cardiac troponin-I target molecule - as optimal candidates. Upon synthesis and characterization, these formulations exhibited distinct rebinding patterns (Figure 22), desirable hydrodynamic particle sizes (Figure 21: A, C, E), and excellent colloidal stability (Figure 21: B, D, F).

### 1. Experimental procedure of ML-based MIP-NP formulations' synthesis

Bare glass beads with a diameter of 0.75-1.00 mm, used for functionalization and peptides' immobilization, were purchased from Carl Roth (Karlsruhe, Germany). All functional monomers 2-methacryloyloxyethyl phosphate (MEP), 2,6-bis(acrylamido)pyridine (BAAPy), N,O-bismethacryloyl ethanolamine (NOBE), N-(3-aminopropyl)methacrylamide hydrochloride (APMA), 2-acrylamido-2-methyl-1-propanesulfonic acid (AMPSA), N-isopropylacrylamide (NIPAm), and crosslinker (N,N' methylenebisacrylamide, MBA), alongside N'-tetramethylethylenediamine (TEMED), 1-ethyl-3-(3-dimethylaminopropyl) carbodiimide (EDC), N-hydroxy-succinimide (NHS), ammonium persulphate (APS), bovine serum albumin (BSA), ethanolamine and phosphate-buffered saline (PBS) were purchased from Sigma Aldrich (Steinheim, Germany). PBS solution (10 mM) was used as buffer. The hexaferrocyanide (K3[Fe(CN)6]), ultra-pure water, methanol (MeOH), ethanol (EtOH), and syringe filters of 0.45 µm pore size were purchased from Carl Roth (Karlsruhe, Germany). Moreover, sodium hydroxide (NaOH) was purchased from VWR (Dresden, Germany), while acryloxyethyl thiocarbamoyl Rhodamine B (RhB monomer) was obtained from Polysciences Europe GmbH (Hirschberg an der Bergstrasse, Germany). All chemicals and solvents were high-performance liquid chromatography (HPLC)/analytical graded and were used without any further purification. The template molecule sequences (YEVHHQKLVFFA, SEQ ID NO: 3, and ISASRKLQLK, SEQ ID NO: 4,) were synthesized by TU Berlin.

During synthesis, a vacuum oven (Vacutherm, Fischer Scientific GmbH, Germany) was used for drying purposes, alongside a bath sonicator (Bandelin Sonorex Digiplus, Eydam GmbH, Germany) for ensuring that monomers are uniformly distributed throughout the solution. The hydrodynamic size distribution and Zeta potential of synthesized nanoMIPs were characterized by using dynamic light scattering (DLS) and a Zetasizer (Zetasizer Pro, Malvern Panalytical Ltd, United Kingdom) at a temperature of 25 ± 0.1°C. Additionally, MarvinSketch 23.14 software (ChemAxon. (2023). MarvinSketch 23.14. https://chemaxon.com/products/marvin) from ChemAxon was used to analyze the primary structure of both peptides, conducting elemental analysis and extracting their molecular weights for further implementations. Fluorescent microscopy (BZ-X810 coupled with BZ-X800 Analyzer software, Keyence Corporation, Japan) was employed to take fluorescent images under 10× magnification. The peptides' rebinding capabilities against the synthesized nanoMIPs were investigated via voltammetric measurements using a compact electrochemical setup and GSPE (PalmSens4 Potentiostatic and PS Trace 5.9 software, PalmSens B.V., The Netherlands).

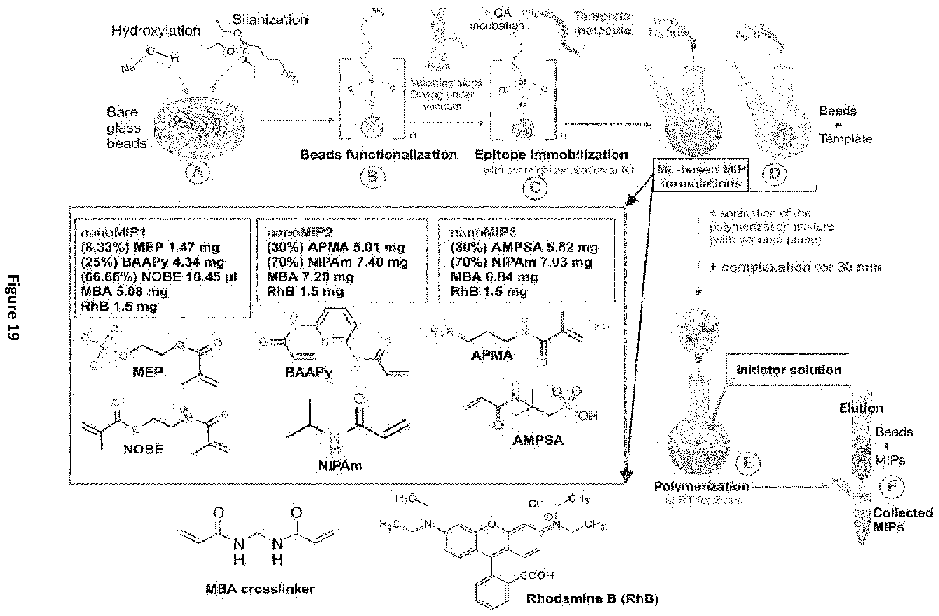

The nanoMIPs synthesis process (Figure 19) starts by weighting approximately 60 g of bare glass beads, as solid support for peptide immobilization. Prior to immobilization, the beads were functionalized with 2 M NaOH via boiling process (100-120°C) for 15 min. This step ensures that hydroxyl (-OH) groups attached (covalently) onto the beads' surface. The NaOH incubation also increases the hydrophilicity of the glass surface. The final pH was adjusted (when needed) with additional washing steps. The pH for nanoMIP1 was 6.8, while for nanoMIP2 and nanoMIP3 the pH was 6.4 and 6.7, respectively. Upon 1 hr drying process under vacuum, the NaOH-beads were transferred into a round-bottom flask and subjected to 30 minutes N2 flow with inlet and outlet, to get rid of the O2 molecules. Subsequently, beads were incubated overnight with 1 ml of 3-aminopropyl)triethoxysilane (APTES) in 47 ml anhydrous toluene. Since toluene is not miscible in water, the following washing steps were considered using acetone (4 x 50 ml) and methanol (2 x 50 ml).

After the glass beads have been functionalized with NaOH (hydroxylation) and APTES (silanization), the next step involved incubating them with 13.44 ml glutaraldehyde (GA) dissolved in 34.56 ml PBS, for 2 hrs at room temperature (RT). The two aldehyde groups react with primary amine groups (-NH₂) present on the APTES-functionalized glass surface. This reaction promotes effective crosslinking of the amine groups onto the surface, via forming a covalent imine (C=N) bond. The use of filtered PBS as solvent maintains a consistent pH and mimics physiological conditions similar to those found in biological systems. Upon GA incubation, approximately 10 mg of peptide - amyloid-β (for nanoMIP1) and cardiac troponin-I (for nanoMIP2 and 3), respectively - dissolved in 40 ml PBS was incubated overnight at RT. Here, all the washing processes of the beads, after each functionalization, were carried out using 50 ml deionized (DI) water, in multiple steps - typically five. The beads' drying steps involved the use of a vacuum pump for 1 hr.

Considering that vacuuming might induce strong suction forces that can disrupt and/or damage the peptide chains from the functionalized surfaces, the following washing steps were carried out with 50 ml DI water (5 times) via pipetting. Pipetting ensures that the washing processes are more controlled, while protecting the functional groups on the peptides. For the Schiff base reduction, to stabilize the secondary amine bonds (imine bonds can be hydrolytically unstable) and therefore stabilizing the immobilized peptides, we incubated the beads for 30 min at RT with 35 mg of sodium borohydride (NaBH₄) in 35 ml PBS. Afterwards, washing steps (5x) via pipetting were considered using 50 ml DI water. Despite the polymerization mixture primarily containing only MIP components, there could still be sites on the beads or partially polymerized structures that might cause non-specific binding. Moreover, since we functionalized the beads, prior to template immobilization, with GA, this could lead to non-specific interactions. Hence, to saturate these potential sites and to ensure that template molecules only interact with the monomers/crosslinker, we incubated the beads for 15 min at RT with 500 µl ethanolamine of 10 mM dissolved in 49.5 ml filtered PBS. Lastly, beads were washed 5 times with 50 ml DI water, and left to dry for 1 hr under vacuum.

The dried beads and, separately, the polymerization mixture were then subjected to purging for 30 min. Upon purging, the polymerization mixture (detailed in Figure 19) was considered for sonication with vacuuming for 20 min, to thoroughly mix the solution and to remove other gases that could inhibit the polymerization process. The sonicating process basically breaks up the gas bubbles, while vacuuming removes them efficiently via suction. Subsequently, the polymerization solution was transferred onto the beads, slowly and evenly, using syringe and needle; all under N₂-filled balloons, and subjected to complexation for 30 min at RT. The use of N₂-filled balloons ensures an inert atmosphere around the solution/beads, preventing O₂ from impeding the reactions. Ultimately, the initiator solution (1% w/v) was prepared by dissolving 1.09 mg of ammonium persulphate (APS) and 3 µl of N,N,N',N'-tetramethylethylenediamine (TEMED) in a total volume of 1 ml. This solution was then transferred onto the beads coated with the polymerization mixture. The polymerization process lasted for 2 hrs at RT before being terminated.

Prior to collecting the nanoMIPs, the polymerization mixture containing unbound species was used for transferring all the beads into a column with filter, and then discarded. The nanoMIPs were then collected via sequential hot-wash elutions, leaving the beads and template molecules behind, with hot-wash temperatures ranging between 73.8-75 °C depending on the specific synthesis. Each polymerization solution (for nanoMIP1, 2, and 3) was prepared in a total volume of 50 ml milli-Q water.

The preparation steps were as follows:
(i) For nanoMIP1, 45 ml of water was poured in a round-bottom flask, filled-up completely upon monomers/crosslinker/RhB weighted using a microbalance. The remaining solvent (5x1 ml of water or EtOH) was used to collect the weighted quantities.
(ii) For both nanoMIP2 and nanoMIP3, 46 ml was initially poured in round-bottom flasks, filled up to a total 50 ml volume after the monomers/crosslinker/RhB were weighted and properly dissolved (4x1 ml of water or EtOH).

Hydrodynamic size and surface charge measurements (Stetefeld, J., McKenna, S. A., & Patel, T. R. (2016). Dynamic light scattering: a practical guide and applications in biomedical sciences. Biophysical Reviews, 8(4), 409-427. DOI: 10.1007/s12551-016-0218-6; Bhattacharjee, S. (2016). DLS and zeta potential - What they are and what they are not?. Journal of Controlled Release, 235, 337-351. DOI: 10.1016/j.jconrel.2016.06.017) were conducted after 3.5 ml of synthesized nanoMIPs were sonicated for 25 min at 20-25 °C, and filtered using 0.45 µm pore diameter filters. We considered the sonication process to disperse imminent nanoparticle aggregates and to ensure a uniform suspension of the nanoMIPs, while the filtering step removed the remaining larger aggregates and potential impurities. The samples volume used in the capillary cell (Malvern cuvette) was 700 µl. The equilibration time was set for 200 s at 25 °C, and 3 measurements, for both DLS and Zeta potential, were recorder during each acquisition.

The (re)binding characteristics of nanoMIPs towards each epitope were examined using GSPEs and SWV method (Mirceski, V., Komorsky-Lovrić, Š., & Lovrić, M. (2007). Square-wave voltammetry. Electroanalysis, 19(22), 2411-2422. DOI: 10.1002/elan.200703916). The GSPE surfaces were prepared a few days prior to sensor development by incubating the gold surfaces with drop-casted 30 µl of 2 mM mercaptoundecanoic acid (MUDA). MUDA is commonly used to modify gold surfaces through the formation of a self-assembled monolayer, where thiol groups bind to the gold, creating stable coatings. During incubation, since MUDA is light sensitive, the gold surfaces were covered with aluminum foil. Upon surfaces functionalization and prior to use, the GSPEs were washed 1 time with HPLC EtOH and DI water. The MUDA-coated surfaces and all subsequent signals were recorded using a 10 mM redox marker solution (K₃[Fe(CN)]₆ with 0.1 M potassium chloride - KCI - in DI water).

Since MUDA contains a carboxylic acid (-COOH) group that is not very reactive on its own, 1-ethyl-3-(3-dimethylaminopropyl)carbodiimide)/N-hydroxysuccinimide (EDC/NHS) coupling (Sam, S., Touahir, L., Salvador Andresa, J. R., Allongue, P., Chazalviel, J. N., Gouget-Laemmel, A. C., ... & Boukherroub, R. (2010). Semiquantitative study of the EDC/NHS activation of acid terminal groups at modified porous silicon surfaces. Langmuir, 26(2), 809-814) was used to covalently immobilize nanoMIPs onto the MUDA-coated surface. Therefore, 40 µl of EDC/NHS were drop-casted onto GSPE and incubated for 15 min. Importantly, the volume used for all incubation steps, washings, and signal readouts was maintained at 40 µl for consistency. The hot-washed nanoMIPs were then incubated for 30 min, followed by one washing step with filtered PBS. In order to block any potential non-specific interactions, the GSPE was incubated with bovine serum albumin (BSA) solution (5 mg/mL dissolved in PBS) and ethanolamine solution (1 mM in PBS, pH 8.5) for 5 min each, with a PBS wash between each incubation. The reference signal was then recorded using redox marker solution after incubating the nanoMIPs-GSPE with PBS for 15 min. Different concentrations of analyte solutions (diluted in filtered PBS) were then incubated consecutively for 15 min each, starting from the lowest concentration. The peptide range covered concentrations from 100 nM to 2 µM (100 nM, 200 nM, 400 nM, 800 nM, 1200 nM, 1600 nM, and 2000 nM). After each incubation, the GSPE surface was washed with PBS one time, and upon adding the redox marker solution the surface was thoroughly washed two times (2x10 times up-down pipetting) via drop-casting 40 µl PBS.

Voltammetry readings were recorded in triplicate, and the average was taken as readout signals per each peptide concentration. Finally, the relative signals - representing the sensor response - with respect to the reference were calculated according to literature (Sehit E, Drzazgowska J, Buchenau D, Yesildag C, Lensen M, Altintas Z. Ultrasensitive nonenzymatic electrochemical glucose sensor based on gold nanoparticles and molecularly imprinted polymers. Biosens Bioelectron. 2020. DOI: 10.1016/j.bios.2020.112432). The herein presented methodology, including DLS, Zeta potential and electrochemical sensors' development, was applied consistently with the same parameters for all three nanoMIP formulations.

### 2. Results and discussion

The experimental results for ML-based MIP-NP formulations show that the size of nanoMIPs is critical for ensuring their effectiveness across various applications, and our ML-designed nanoMIPs (Figure 21) demonstrate sizes tailored to these needs. For drug delivery and diagnostic systems, nanoMIPs typically require hydrodynamic sizes between 10-200 nm, as smaller particles facilitate cellular uptake and enhanced circulation. The ML-based nanoMIPs 1 and 2, with sizes of 125.6 nm, fall within this optimal range, aligning with requirements for these applications. For tissue engineering, where structural stability is essential, nanoMIPs may range from 100-500 nm or larger, and nanoMIP3 with a size of 229.8 nm, perfectly matches these specifications. Similarly, biosensing applications demand sizes ranging from 50 nm to several micrometers to maximize surface-area-to-volume ratios for signal detection, correlating well with the sizes of nanoMIPs 1, 2, and 3.

Colloidal stability is another critical parameter for nanoMIPs, ensuring particle dispersion and preventing aggregation, which can compromise performance. Zeta potential values of ±30 mV or higher are considered optimal for achieving sufficient electrostatic repulsion between particles. Our ML-based nanoMIPs demonstrate excellent colloidal stability (Figure 21: B, D, F), with zeta potentials of -40 mV (nanoMIP1), -23 mV (nanoMIP2), and -32 mV (nanoMIP3). Additionally, for biocompatible materials, moderately negative zeta potentials (-10 mV to -40 mV) are ideal to maintain stability without triggering immune responses. All three nanoMIPs meet these criteria, ensuring their suitability for biomedical applications such as drug delivery, tissue engineering, and diagnostics.

The binding behavior of nanoMIPs is essential for their functionality in targeted delivery and diagnostics, and our ML-designed nanoMIPs showcase robust and varied performance (Figure 22). NanoMIP1 exhibits a complex, non-uniform binding pattern, well-explained by nonlinear Freundlich and Langmuir-Freundlich models (R²=0.921), suggesting heterogeneous adsorption sites. NanoMIP2 demonstrates a simpler, more uniform binding behavior with an almost perfect linear correlation (R²=0.99), indicating straightforward kinetics suitable for predictable applications. NanoMIP3 also shows complex binding behavior with strong nonlinear correlations (R²=0.931), aligning with nanoMIP1 in terms of heterogeneous site interactions. Together, these findings highlight that while nanoMIP2 offers predictable, uniform performance, nanoMIP1 and nanoMIP3 are better suited for applications requiring sophisticated, multilayer adsorption mechanisms.

### 3. ML algorithm based on Random Forest classifier used to predict new MIP-NPs

An exemplary ML algorithm that corresponds to Figure 15 can be used for designing MIP-NP formulations specific for new template sequences; the MIP-NPs owning characteristics and properties as in the experimental proof-of-concept validation of the ML algorithm from Example 2 (above),is shown as follows:

import pandas as pd
import numpy as np
rom sklearn.preprocessing import LabelEncoder
from sklearn.model_selection import train_test_split
rom sklearn.ensemble import RandomForestClassifier
from sklearn.metrics import classification_report
   df = pd.read_csv('dataset.csv', decimal=',')

numerical_columns = [
   'T-FMRatio', 'BindingAffinity', 'SolventExposureTemplate', 'TemplateGyrationValue',
'ComplexGyrationValue', 'T-FMMinimumDistance', 'T-FMH-bonds',
   'T-FMLennard-JonesPotential', 'T-FMCoulombicPotential', 'T-
FMTotalInteractionEnergy', 'SimulationTemperatureK', 'SimulationTimeNS',
'DLSsize25C', 'DLSsize38C', 'ZetaPotential',
   'FT-IRPeak1', 'FT-IRPeak2', 'FT-IRPeak3', 'LODRebinding', 'TemperatureMIPsWash'
]
for col in numerical_columns:
   df[col] = df[col].astype(str).replace(',', '.').astype(float)

categorical_columns = [
   'ComplexID', 'TemplateID', 'TemplateSS', 'MonomerID', 'MonomerName',
'MonomerType',
   'MonomerFormula', 'Crosslinking', 'Crosslinker', 'OutcomeID', 'FingerprintPeak1',

'FingerprintPeak2', 'FingerprintPeak3', 'LODUnit', 'OutcomeEXP',
'Outcome_availability'
]
label_encoders = {col: LabelEncoder() for col in categorical_columns}
for col in categorical_columns:
    df[col] = label_encoders[col].fit_transform(df[col].astype(str))

def count_residue_types(sequence):
    sequence = sequence.upper().strip()
    if not sequence:
            return [0, 0, 0, 0, 0, 0, 0, 0]
    basic = sum(1 for aa in sequence if aa in 'RHK')
    acidic = sum(1 for aa in sequence if aa in 'DE')
    neutral = sum(1 for aa in sequence if aa in 'STCAGNPQILMFVWY')
    polar = sum(1 for aa in sequence if aa in 'STNQDEHRKC')
    non_polar = sum(1 for aa in sequence if aa in 'GAVLIMPWFY')
    glycine = sequence.count('G')
    proline = sequence.count('P')
    cysteine = sequence.count('C')
    return basic, acidic, neutral, polar, non_polar, glycine, proline, cysteine

df['TemplateSequence'] = df['TemplateSequence'].fillna('').astype(str)
counts = df['TemplateSequence'].apply(count_residue_types)
#print(counts.head())
df[['basic_count', 'acidic_count', 'neutral_count', 'polar_count', 'non_polar_count',
'glycine_count', 'proline_count', 'cysteine_count']] = pd.DataFrame(counts.tolist(),
index=df.index)

def calculate_predicted_performance (row):
    weight_T_FMMinimumDistance = 0.5
    weight_T_FMH_bonds = 0.8
    weight_T_FMTotalInteractionEnergy = 1.9

if (row['MonomerType'] == 0 and (row['acidic_count'] > 0 or row['polar_count'] > 0)
and
row['ComplexGyrationValue'] >= 1.0 and row['T-FMMinimumDistance'] *
weight_T_FMMinimumDistance <= 0.4 and row['T-FMH-bonds'] *
weight_T_FMH_bonds >=
300 and row['T-FMTotalInteractionEnergy'] * weight_T_FMTotalInteractionEnergy <=
-40):
return 'Good'
elif (row['MonomerType'] == 1 and (row['basic_count'] > 0 or row['polar_count'] > 0)
and
row['ComplexGyrationValue'] >= 1.0 and row['T-FMMinimumDistance'] *
weight_T_FMMinimumDistance <= 0.4 and row['T-FMH-bonds'] *
weight_T_FMH_bonds >=
300 and row['T-FMTotalInteractionEnergy'] * weight_T_FMTotalInteractionEnergy <=
-40):
return 'Good'
elif (row['MonomerType'] == 3 and row['non_polar_count'] > 0 and
row['ComplexGyrationValue'] >= 1.0 and row['T-FMMinimumDistance'] *
weight_T_FMMinimumDistance <= 0.4 and row['T-FMH-bonds'] *
weight_T_FMH_bonds >=
300 and row['T-FMTotalInteractionEnergy'] * weight_T_FMTotalInteractionEnergy <=
-40):
return 'Good'
elif (row['T-FMMinimumDistance'] * weight_T_FMMinimumDistance > 0.4 and
row['T-FMH-bonds'] * weight_T_FMH_bonds < 800 and row['T-
FMTotalInteractionEnergy'] *
weight_T_FMTotalInteractionEnergy >= -40):
return 'Bad'
else:
return 'Optimal'

df['Performance'] = df.apply(calculate_predicted_performance, axis=1)

feature_columns = ['T-FMRatio', 'BindingAffinity', 'SolventExposureTemplate',
'TemplateGyrationValue', 'ComplexGyrationValue', 'T-FMMinimumDistance', 'T-FMH-
bonds',
   'T-FMLennard-JonesPotential', 'T-FMCoulombicPotential', 'T-
FMTotalInteractionEnergy',
'DLSsize25C', 'DLSsize38C', 'ZetaPotential',
   'FT-IRPeak1', 'FT-IRPeak2', 'FT-IRPeak3', 'LODRebinding', 'basic_count',
'acidic_count',
'neutral_count', 'polar_count', 'non_polar_count', 'glycine_count', 'proline_count',
'cysteine_count']
X = df[feature_columns]
y = df.apply(calculate_predicted_performance, axis=1)

label_encoder = LabelEncoder()
y_encoded = label_encoder.fit_transform(y)

X_train, X_test, y_train, y_test = train_test_split(X, y_encoded, test_size=0.3,
random_state=135)
model = RandomForestClassifier(n_estimators=400, max_depth=10,
random_state=135)
model.fit(X_train, y_train)
y_pred = model.predict(X_test)

print(classification_report(y_test, y_pred, target_names=label_encoder.classes_))

df.to_csv('Dataset_with_Predictions.csv', index=False)
df = pd.read_csv('Dataset_with_Predictions.csv', decimal=',')

numerical_columns = [
   'T-FMRatio', 'BindingAffinity', 'SolventExposureTemplate', 'TemplateGyrationValue',
'ComplexGyrationValue', 'T-FMMinimumDistance',

'T-FMH-bonds', 'T-FMLennard-JonesPotential', 'T-FMCoulombicPotential', 'T-
FMTotalInteractionEnergy', 'SimulationTemperatureK',
'SimulationTimeNS', 'DLSsize25C', 'DLSsize38C', 'ZetaPotential', 'FT-IRPeak1', 'FT-
IRPeak2',
'FT-IRPeak3', 'LODRebinding',
'TemperatureMIPsWash', 'basic_count', 'acidic_count', 'neutral_count',
'polar_count',
'non_polar_count', 'glycine_count', 'proline_count', 'cysteine_count'
]

for col in numerical_columns:
df[col] = df[col].astype(str).str.replace(',', '.')
df[col] = pd.to_numeric(df[col], errors='coerce')

df['Performance'] = df['Performance'].astype('category')

def select_best_fm(df, fm_type, aa_type, count, peptide_length,
performance_range=0.5):
if fm_type == 'acidic':
filtered_df = df.loc[(df['MonomerType'] == 0) & ((df[aa_type] > 0) |
(df['polar_count'] >
0))]
elif fm_type == 'basic':
filtered_df = df.loc[(df['MonomerType'] == 1) & ((df[aa_type] > 0) |
(df['polar_count'] >
0))]
elif fm_type == 'neutral':
filtered_df = df.loc[(df['MonomerType'] == 3) & ((df[aa_type] > 0) |
(df['non_polar_count']
> 0))]
else:
return None, None

filtered_df.loc[:, 'T-FMTotalInteractionEnergy'] = pd.to_numeric(filtered_df['T-
FMTotalInteractionEnergy'], errors='coerce')
filtered_df.loc[:, 'T-FMTotalInteractionEnergy'] = filtered_df['T-
FMTotalInteractionEnergy'].astype(float)

min_energy = filtered_df['T-FMTotalInteractionEnergy'].min()
threshold_value = float(min_energy + abs(min_energy * performance_range))

good_fms = filtered_df.loc[(filtered_df['Performance'] == 'Good') & (filtered_df['T-
FMTotalInteractionEnergy'] <= threshold_value)]
optimal_fms = filtered_df.loc[(filtered_df['Performance'] == 'Optimal') &
(filtered_df['T-
FMTotalInteractionEnergy'] <= threshold_value)]

if not good_fms.empty:
good_fms = good_fms.sample(frac=1).reset_index(drop=True)
selected_good_fm = good_fms.iloc[0]
else:
selected_good_fm = None

if not optimal_fms.empty:
optimal_fms = optimal_fms.sample(frac=1).reset_index(drop=True)
selected_optimal_fm = optimal_fms.iloc[0]
else:
selected_optimal_fm = None

return selected_good_fm, selected_optimal_fm

def map_monomers_to_aa_types(df, aa_counts, peptide_length):
basic_count, acidic_count, neutral_count, polar_count, non_polar_count,
glycine_count,

proline_count, cysteine_count = aa_counts

   best_acidic_fm, acidic_fm_optimal = select_best_fm(df, 'acidic', 'basic_count',
basic_count, peptide_length)
   best_basic_fm, basic_fm_optimal = select_best_fm(df, 'basic', 'acidic_count',
acidic_count,
peptide_length)
   best_neutral_fm, neutral_fm_optimal = select_best_fm(df, 'neutral',
'neutral_count',
neutral_count, peptide_length)

   acidic_ratio_good = basic_count
   basic_ratio_good = acidic_count
   neutral_ratio_good = neutral_count

   acidic_ratio_optimal = basic_count
   basic_ratio_optimal = acidic_count
   neutral_ratio_optimal = neutral_count

   return (best_acidic_fm, acidic_ratio_good, best_basic_fm, basic_ratio_good,
best_neutral_fm, neutral_ratio_good,
         acidic_fm_optimal, acidic_ratio_optimal, basic_fm_optimal,
basic_ratio_optimal,
neutral_fm_optimal, neutral_ratio_optimal)

def get_crosslinker_info(df):
   crosslinkers_df = df[df['Crosslinking'] == 1]
   if crosslinkers_df.empty:
         return None, None

   crosslinkers_df.loc[:, 'T-FMTotalInteractionEnergy'] =
pd.to_numeric(crosslinkers_df['T-

FMTotalInteractionEnergy'], errors='coerce')
    crosslinkers_df.loc[:, 'T-FMTotalInteractionEnergy'] = crosslinkers_df['T-
FMTotalInteractionEnergy'].astype(float)

    crosslinkers = crosslinkers_df.sort_values(by='T-FMTotalInteractionEnergy',
ascending=True).iloc[0]

    crosslinker_ratio = crosslinkers['T-FMRatio']

    return crosslinkers, crosslinker_ratio

def display_results(acidic_good, acidic_good_ratio, basic_good, basic_good_ratio,
neutral_good, neutral_good_ratio,
            acidic_optimal, acidic_optimal_ratio, basic_optimal, basic_optimal_ratio,
neutral_optimal, neutral_optimal_ratio,
            crosslinkers, crosslinker_ratio):
    print("FM names with types and ratios (Good):")
    if acidic_good is not None:
            print(f"Acidic (Good): {acidic_good['MonomerName']} (Type:
{acidic_good['MonomerType']},
Ratio: {acidic_good_ratio})")
    if basic_good is not None:
            print(f"Basic (Good): {basic_good['MonomerName']} (Type:
{basic_good['MonomerType']},
Ratio: {basic_good_ratio})")
    if neutral_good is not None:
            print(f"Neutral (Good): {neutral_good['MonomerName']} (Type:
{neutral_good['MonomerType']}, Ratio: {neutral_good_ratio})")

    print("\nFM names with types and ratios (Optimal):")
    if acidic_optimal is not None:
            print(f"Acidic (Optimal): {acidic_optimal['MonomerName']} (Type:

{acidic_optimal['MonomerType']}, Ratio: {acidic_optimal_ratio})")
    if basic_optimal is not None:
        print(f"Basic (Optimal): {basic_optimal['MonomerName']} (Type:
{basic_optimal['MonomerType']}, Ratio: {basic_optimal_ratio})")
    if neutral_optimal is not None:
        print(f"Neutral (Optimal): {neutral_optimal['MonomerName']} (Type:
{neutral_optimal['MonomerType']}, Ratio: {neutral_optimal_ratio})")

    if crosslinkers is not None:
        print(f"\nCrosslinking: TRUE, Ratio: {crosslinker_ratio}")
    else:
        print("\nCrosslinking: FALSE")

def get_user_sequence():
    user_input = input("Enter a new template amino acid sequence (in one-letter AA
codes):
").upper()
    return user_input

def count_residue_types(sequence):
    sequence = sequence.upper().strip()
    if not sequence:
        return [0, 0, 0, 0, 0, 0, 0, 0]
    basic = sum(1 for aa in sequence if aa in 'RHK')
    acidic = sum(1 for aa in sequence if aa in 'DE')
    neutral = sum(1 for aa in sequence if aa in 'STCAGNPQILMFVWY')
    polar = sum(1 for aa in sequence if aa in 'STNQDEHRKC')
    non_polar = sum(1 for aa in sequence if aa in 'GAVLIMPWFY')
    glycine = sequence.count('G')
    proline = sequence.count('P')
    cysteine = sequence.count('C')
    return basic, acidic, neutral, polar, non_polar, glycine, proline, cysteine

def main():
   df_predictions = pd.read_csv('Dataset_with_Predictions_on_FMs.csv', decimal=',')

   new_sequence = get_user_sequence()
   peptide_length = len(new_sequence)

   aa_counts = count_residue_types(new_sequence)

   (best_acidic_fm, acidic_ratio_good, best_basic_fm, basic_ratio_good,
best_neutral_fm,
neutral_ratio_good,
   acidic_fm_optimal, acidic_ratio_optimal, basic_fm_optimal, basic_ratio_optimal,
neutral_fm_optimal, neutral_ratio_optimal) =
map_monomers_to_aa_types(df_predictions,
aa_counts, peptide_length)

   crosslinkers, crosslinker_ratio = get_crosslinker_info(df_predictions)

   display_results(best_acidic_fm, acidic_ratio_good, best_basic_fm,
basic_ratio_good,
best_neutral_fm, neutral_ratio_good,
         acidic_fm_optimal, acidic_ratio_optimal, basic_fm_optimal,
basic_ratio_optimal,
neutral_fm_optimal, neutral_ratio_optimal,
         crosslinkers, crosslinker_ratio)

if __name__ == "__main__":
   main()

## Claims

1. A computer-implemented method for producing a multifunctional molecularly imprinted polymer nanoparticle based on a machine learning algorithm, wherein the molecularly imprinted polymer nanoparticle comprises a template molecule, a functional monomer and a cross-linker, wherein the computer-implemented method comprises:
(S11) receiving an input dataset;
(S12) importing a programming library;
(S13) processing a pre-defined dataset, wherein the pre-defined dataset comprises data of functional monomers, template molecules and cross-linkers;
(S14) finding a functional monomer, a template molecule and a cross-linker, wherein the functional monomer, the template molecule and the cross-linker have a maximum number of H-bonds and a minimum value of total interaction energy based on the input dataset; and
(S15) outputting data of at least one of the followings: the functional monomer, the template molecule and the cross-linker on a display means.

2. The computer-implemented method according to claim 1, wherein after the (S13) processing a pre-defined dataset, the computer-implemented method further comprises:
prompting a user on the display means to add data of a new functional monomer into the predefined dataset via an input means;
preferably the data of the new functional monomer comprises at least one of the followings: an index number, a name, a type, a docking value, an epitope sequence, a contact number, a solvent-accessible surface area, a H-bonds number, and a total interaction energy of the new functional monomer.

3. The computer-implemented method according to claim 1 or 2, wherein after the (S13) processing a pre-defined dataset, the computer-implemented method further comprises:
screening the pre-defined dataset on the display means; and/or
after the (S14) finding a functional monomer, and a cross-linker, the computer-implemented method further comprises: storing the data of the at least one of the functional monomer, the template molecule and the cross-linker to a new column of the pre-defined dataset.

4. The computer-implemented method according to any one of claims 1 to 3, wherein the data of the at least one of the template molecule, the functional monomer and the cross-linker comprises at least one of the followings: an index number, a name, a type, a docking value, an epitope sequence, a solvent-accessible surface area, a H-bonds number, and a total interaction energy of the at least one of the template molecule, the functional monomer and the cross-linker.

5. The computer-implemented method according to any one of claims 1 to 4, wherein in the (S14) finding a functional monomer and a cross-linker, a machine learning classifier module is used; preferably, Random Forest classifier is used; and/or
wherein all the steps of the computer-implemented method are repeated for all data in a training dataset and a testing dataset for training; and/or
a confusion matrix is calculated to assess a prediction accuracy of the computer-implemented method.

6. The computer-implemented method according to any one of claims 1 to 5, wherein the input dataset comprises data relating to at least two of the followings: a template molecule, a functional monomer and a cross-linker; preferably, input dataset comprises a binding affinity.

7. The computer-implemented method according to any one of claims 1 to 6, wherein the functional monomer is temperature-responsive for the molecularly imprinted polymer nanoparticle to release an active ingredient to a target; and/or the template molecule is DDGCEPCGNDT (SEQ ID NO: 2) peptide, extracted from the DNase-I structure; and/or YEVHHQKLVFFA (SEQ ID NO: 3) and/or ISASRKLQLK (SEQ ID NO: 4), and/or an active ingredient to be delivered to a target by the molecularly imprinted polymer nanoparticle is a drug or a mRNA; and/or a target where an active ingredient is to be delivered by the molecularly imprinted polymer nanoparticle is a cell, preferably a lung cell.

8. A method for producing a multifunctional molecularly imprinted polymer nanoparticle based on a machine learning algorithm, wherein the molecularly imprinted polymer nanoparticle comprises a template molecule, a functional monomer and a cross-linker, wherein the method comprises:
(S21) selecting an input dataset based on an intended use;
(S22) inputting the input dataset via an input means;
(S23) receiving a data of at least one of the followings: a functional monomer and a cross-linker on a display means, wherein the data is obtained according to any one of claims 1 to 7; and
(S24) producing the molecularly imprinted polymer nanoparticle according to the data of at least one of the followings: the template molecule, the functional monomer and the cross-linker.

9. The method according to claim 8, wherein before the (S21) selecting an input dataset based on an intended use or before the (S22) inputting the input dataset via an input means, the method further comprises converting a pre-defined dataset into a machine-readable format; preferably the machine-readable format is CSV format.

10. An apparatus for producing a multifunctional molecularly imprinted polymer nanoparticle based on a machine learning algorithm, wherein the molecularly imprinted polymer nanoparticle comprises a template molecule, a functional monomer and a cross-linker, wherein the apparatus comprises:
a receiving means configured to receive an input dataset,
an input means configured for a user to input data of a new template molecule ,
a processing means configured to import a programming library, and/or process a pre-defined dataset, and/or find a functional monomer, and a cross-linker, wherein the functional monomer, the template molecule and the cross-linker have a maximum number of H-bonds and a most negative value of total interaction energy based on the input dataset, and
a display means configured to screen a pre-defined dataset and/or output data of at least one of the followings: the template molecule, the functional monomer and the cross-linker.

11. An electronic device, wherein the electronic device comprises a processor and a memory, the memory stores a computer-executable instruction executable by the processor, the processor is configured to execute the computer-executable instruction so as to implement a computer-implemented method for producing a multifunctional molecularly imprinted polymer nanoparticle based on a machine learning algorithm, wherein the molecularly imprinted polymer nanoparticle comprises a template molecule, a functional monomer and a cross-linker, according to any one of claims 1 to 7.

12. A computer-readable storage medium, wherein the computer-readable storage medium stores a computer-executable instruction, that, when being executed by a processor, causes the processor to implement a computer-implemented method for producing a multifunctional molecularly imprinted polymer nanoparticle based on a machine learning algorithm, wherein the molecularly imprinted polymer nanoparticle comprises a template molecule, a functional monomer and a cross-linker, according to any one of claims 1 to 7.

13. A multifunctional molecularly imprinted polymer nanoparticle, wherein the molecularly imprinted polymer nanoparticle comprises a template molecule, a functional monomer and a cross-linker; preferably, the molecularly imprinted polymer nanoparticle is produced by a method according to any one of claims 1 to 7.

14. A molecularly imprinted polymer nanoparticle for use in treating cystic fibrosis, wherein the molecularly imprinted polymer nanoparticle comprises a template molecule, a functional monomer and a cross-linker; preferably, the molecularly imprinted polymer nanoparticle is produced by a method according to any one of claims 1 to 7.

15. A pharmaceutical composition comprising an effective amount of a molecularly imprinted polymer nanoparticle according to claim 13 or 14.
